# FASCICULE DE BREVET EUROPEEN

(11) **EP 0 914 437 B1**
(45) Date de publication et mention de la délivrance du brevet: **31.05.2006**
(21) Numéro de dépôt: 97925109.7
(22) Date de dépôt: 20.05.1997
(51) Int. Cl.: C12N 15/31, G01N 33/569, C07K 14/35, C07K 16/12, A61K 39/04

(54) **IDENTIFICATION ET CLONAGE D'UN ANTIGENE MYCOBACTERIEN CORRESPONDANT A UNE HEMAGGLUTININE DE LIAISON A L'HEPARINE**
IDENTIFIZIERUNG UND KLONIERUNG EINES MYCOBAKTERIEN ANTIGENS DER EINER HEPARIN-BINDENDEN HEMAGLUTININ ENTSPRICHT
IDENTIFICATION AND CLONING OF A MYCOBACTERIAL ANTIGEN CORRESPONDING TO A HEPARIN-BINDING HAEMAGGLUTININ

(30) Priorité: 17.05.1996 FR 9606168
(43) Date de publication de la demande: 12.05.1999
(73) Titulaire: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: MENOZZI, Franco, B-7022 Mons-Hyon (BE); LOCHT, Camille, F-59830 Wannehain (FR)
(74) Mandataire: Desaix, Anne
(86) Numéro de dépôt international: PCT/FR1997/000886
(87) Numéro de publication internationale: WO 1997/044463

(56) Documents cités:
- MENOZZI, F. ET AL.: "A heparin - binding hemagglutinin in mycobacteria." ABSTRACTS OF THE GENERAL MEETING OF THE AMERICAN SOCIETY FOR MICROBIOLOGY 95 (0). 1995. PAGE 193, ABSTRACT B-159, XP002041282 & 95TH GENERAL MEETING OF THE AMERICAN SOCIETY FOR MICROBIOLOGY, WASHINGTON, D.C., USA, MAY 21-25, 1995. ,
- BRENNAN, M J. ET AL.: "Identification of a heparin - binding mycobacterial hemagglutinin." JOURNAL OF CELLULAR BIOCHEMISTRY SUPPLEMENT 0 (19B). 1995. PAGE 66. ABSTRACT B3-104, XP002041283 & KEYSTONE SYMPOSIUM ON MOLECULAR MECHANISMS IN TUBERCULOSIS, TAMARRON, COLORADO, USA, FEBRUARY 19-25, 1995. ,
- ISAACS, R. : "Borrelia burgdorferi bind to epithelial cell proteoglycans" JOURNAL OF CLINICAL INVESTIGATION, vol. 93, février 1994, pages 809-819, XP002041419
- MENOZZI, F. ET AL.: "Heparin-inhibitable lectin activity of the filamentous hemagglutinin adhesin of Bordetella pertussis" INFECTION AND IMMUNITY., vol. 62, mars 1994, WASHINGTON US, pages 769-778, XP002041284 cité dans la demande
- HUDSON, M. ET AL.: "Comparison of the fibronectin-binding ability and tumor efficacy of various mycobacteria" CANCER RESEARCH., vol. 50, 1990, MD US, pages 3843-3847, XP002041285
- ROUSE, D. ET AL.: "Immunological characterization of recombinant antigens isolated from a Mycobacterium avium lambdagt11 expression library by using monoclonal antibody probes" INFECTION AND IMMUNITY., vol. 59, août 1991, WASHINGTON US, pages 2595-2600, XP002041286 cité dans la demande
- "Mycobacterium tuberculosis cosmid SCY20G9" BANQUE DE DONN¹ES EMBL, IDENTIFICATEUR MTCY20G9; NUM¹RO D'ACCÈS Z77162 , 18 septembre 1996, XP002041287
- MENOZZI F D ET AL: "Identification of a heparin - binding hemagglutinin present in mycobacteria." JOURNAL OF EXPERIMENTAL MEDICINE, (1996 SEP 1) 184 (3) 993-1001., XP002041288
- PETHE ET AL MOLECULAR MICROBIOLOGY vol. 39, no. 1, 2001, pages 89 - 99

## Description

L'invention concerne des séquences peptidiques permettant l'adhérence de mycobactéries à des cellules hôtes, notamment à des cellules épithéliales. Plus particulièrement, l'invention concerne un antigène mycobactérien de type hémagglutinine de liaison à l'héparine (HBHA) obtenu à partir de *Mycobacterium bovis* BCG ou *Mycobacterium tuberculosis.* L'invention concerne aussi une séquence peptidique recombinante permettant l'adhérence de mycobactéries à des cellules hôtes. L'invention concerne notamment le produit d'expression d'une souche *Escherichia coli* transformée avec une séquence nucléotidique codant pour une protéine permettant l'adhérence de mycobactéries à ces cellules hôtes. Ces polypeptides peuvent être utilisés dans des compositions immunogènes, pour la préparation de vaccins contre les infections mycobactériennes, et pour le diagnostic sérologique d'infections mycobactériennes.

L'invention concerne également une séquence nucléotidique codant pour une séquence peptidique permettant l'adhérence de mycobactéries à des cellules hôtes, et plus particulièrement une séquence nucléotidique codant pour un antigène mycobactérien de type hémagglutinine de liaison à l'héparine (HBHA). L'invention concerne également des vecteurs recombinants comprenant ladite séquence nucléotidique ainsi que l'utilisation de ces vecteurs pour l'obtention d'hôtes cellulaires recombinants pouvant être utilisés en thérapie, notamment en thérapie anticancéreuse.

Les mycobactéries sont parmi les microorganismes pathogènes les plus importants qui causent des maladies aussi bien chez l'homme que chez l'animal. Les infections mycobactériennes sont toujours parmi les premières causes de mortalité dans le monde. La tuberculose humaine, causée par *Mycobacterium tuberculosis,* conduit à elle seule à approximativement 3 millions de morts chaque année (1,2). *Mycobacterium bovis* cause la tuberculose chez les bovins, mais il est également très virulent pour l'homme. La lèpre, causée par *Mycobacterium leprae,* reste un problème majeur de santé non résolu dans les pays en voie de développement (3).

Les infections par des membres du complexe *Mycobacterium avium-intracellulare* causent des maladies chez les oiseaux et le porc et sont parmi les infections opportunistes les plus fréquentes que l'on trouve chez les patients atteints du syndrôme d'immunodéficience acquise (SIDA) (4, 5). En outre, la récente réapparition dramatique de la tuberculose dans les pays développés ainsi que l'apparition et la propagation de souches de *M. tuberculosis* résistantes aux médicaments (6) soulignent la difficulté de contrôler les maladies mycobactériennes.

La caractérisation moléculaire des diverses étapes dans la pathogenèse des maladies mycobactériennes est fondamentale pour le développement d'approches thérapeutiques et prophylactiques rationnelles et optimisées contre ces maladies. Les facteurs de virulence sont souvent de bons antigènes pouvant être utilisés à titre de candidats-vaccins. En dépit de l'importance des infections mycobactériennes, on connaît peu de choses au sujet des mécanismes moléculaires de base impliqués dans leur pathogenèse (7).

Une protéine de 28 kDa a été précédemment isolée à partir d'extraits totaux cellulaires, de préparations membranaires ou de cultures de surnageant de mycobactéries (21, 22). Il a été montré dans ces mêmes publications que cette protéine était capable d'agglutiner les érythrocytes ou auto-agglutiner les mycobactéries. Les analyses par immunoempreintes à l'aide d'anticorps polyclonaux et monoclonaux ont indiqué que cette protéine est différente des protéines du complexe de l'antigène 85 et représente donc une nouvelle protéine. Menozzi et al. décrivent l'identification d'une protéine nommée HBHA et ont montré que des anticorps anti-HBHA étaient capables d'inhiber la fixation de mycobactéries à des cellules épithéliales (23). En revanche, aucun de ces documents ne divulgue d'antigène ou de polypeptide recombinant constitué de la partie C-terminale de HBHA ou de portions de cette partie C-terminale.

L'un des événements initiaux et cruciaux dans la pathogenèse bactérienne est l'adhérence du microorganisme à ses tissus cibles. Les mycobactéries présentent un tropisme pour les macrophages pulmonaires (8). Toutefois, dans la mesure où ces microorganismes sont facilement transmis par aérosol, les premières structures de l'hôte qu'ils rencontrent au cours de l'infection sont celles de l'épithélium respiratoire. Par conséquent, les interactions avec les cellules épithéliales ou avec la matrice extracellulaire (MEC) pendant les étapes initiales et ultérieures de la pathogénèse peuvent être importantes (9), bien qu'elles n'aient pas encore été étudiées de manière poussée.

Dans le contexte de la présente invention, les inventeurs ont obtenu un nouvel antigène mycobactérien impliqué dans l'adhérence de mycobactéries à des cellules hôtes de type épithéliale. Il s'agit de la partie C-terminale d'une hémagglutinine de liaison à l'héparine (heparin binding hemagglutinin ou HBHA) de 28 kDa qui a été obtenue à partir de surnageants de culture et de préparation des parois cellulaires de *Mycobacterium bovis* BCG et de *Mycobacterium tuberculosis.* A partir de cette partie C-terminale, les inventeurs ont pu évaluer et proposer le développement d'une série de polypeptides pouvant être utilisés en diagnostic, en thérapie et en prophylaxie.

L'invention concerne donc une séquence peptidique permettant l'adhérence de mycobacteries à des cellules hôtes, notamment des cellules épithéliales. Plus particulièrement, la séquence peptidique de l'invention est caractérisée en ce qu'il s'agit d'un antigène mycobactérien de type hémagglutinine de liaison à l'héparine (HBHA), notamment un antigène obtenu à partir de *Mycobacterium bovis* BCG ou *Mycobacterium tuberculosis.*

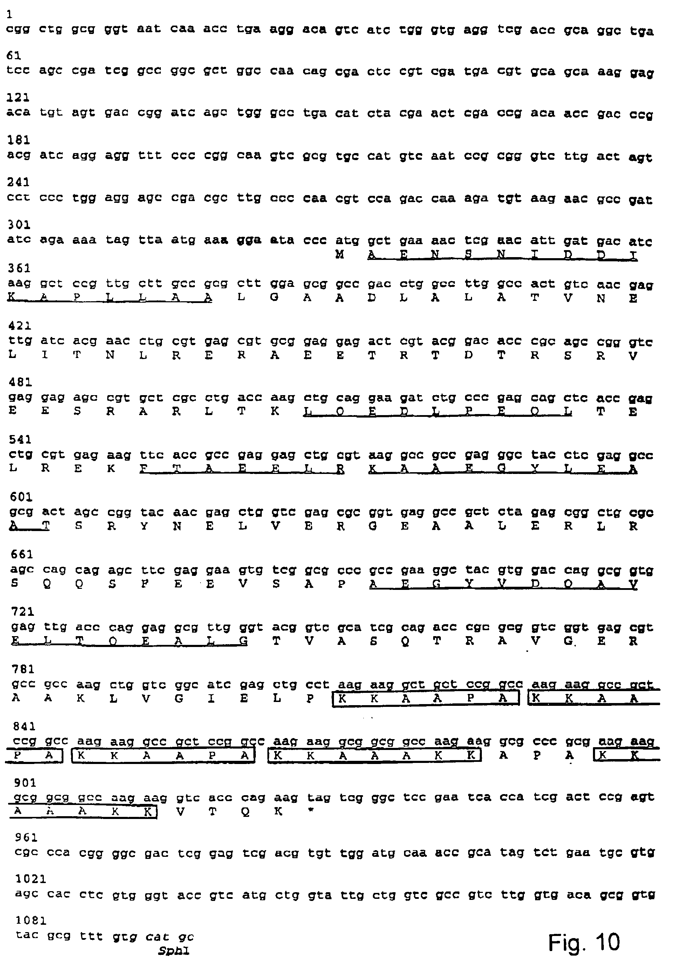

Dans une des réalisations préférées de la présente invention, la séquence peptidique est caractérisée en ce qu'elle consiste en la partie C-terminale de la séquence correspondant à la séquence de la figure 10.

Dans l'ensemble du texte, le terme séquence polypeptidique ou polypeptide représente tout ou partie de la séquence de la figure 10, qui elle-même représente le DNA et la protéine HBHA en tant que telle. Le terme "protéine" désigne de fait la séquence polypeptidique complète modifiée ou non.

De façon préférée, l'invention concerne plus particulièrement une séquence peptidique consistant en une région impliquée dans les interactions avec les glycoconjugués sulfatés et dans la liaison à l'héparine. Cette séquence peptidique est la suivante :

KKAAPAKKAAPAKKAAPAKKAAAKKAPAKKAAAKKVTQK

L'invention concerne donc une séquence peptidique consistant en la portion C-terminale de la séquence de la figure 10 et plus particulièrement les séquences comprenant approximativement les 30 à 50 derniers acides aminés de la portion C-terminale de la séquence de la figure 10. Cette région de la séquence de la figure 10 est impliquée dans l'interaction de la protéine avec l'héparine bien qu'une séquence plus courte, notamment d'environ 10 à 20 acides aminés, puisse être suffisante.

Les inventeurs ont également exprimé la séquence nucléotidique codant pour la séquence peptidique de l'invention chez *E*. *coli.* Le polypeptide obtenu présente un poids moléculaire plus faible que celui de la protéine purifiée à partir de M. *bovis* BCG ou *M. tuberculosis.* Ces différences sont le résultat de modifications post-traductionnelles non réalisées dans *E*. *coli.*

L'invention concerne donc également une séquence peptidique recombinante, caractérisée en ce qu'elle permet l'adhérence de mycobactéries à des cellules hôtes. Plus particulièrement, la séquence recombinante de la présente invention est le produit d'expression d'une séquence nucléotidique codant pour une séquence peptidique permettant l'adhérence de mycobactéries à des cellules hôtes et notamment une séquence antigénique obtenue à partir de M. *bovis* BCG et *M*. *tuberculosis,* ladite séquence recombinante étant par exemple le produit d'expression d'une souche *E*. *coli* transformée avec une séquence nucléotidique appropriée.

L'invention concerne également l'utilisation de l'une des séquences peptidiques décrites précédemment, qu'elle soit recombinante ou non, et plus particulièrement sous sa forme native pour le diagnostic sérologique de la présence de mycobactéries. L'invention concerne également une composition immunogène caractérisée en ce qu'elle comprend l'une des séquences peptidiques décrites précédemment ainsi que l'utilisation de cette séquence peptidique pour la préparation de vaccins contre les infections mycobactériennes, particulièrement les infections causées par *M. bovis* ou *M. tuberculosis.*

Les inventeurs ont également découvert que l'adhérence de mycobactéries à des cellules épithéliales pouvait être inhibée spécifiquement par des glucides sulfatés. L'invention concerne donc l'utilisation d'un glucide sulfaté pour inhiber l'adhérence de mycobactéries à des cellules épithéliales. Les glucides sulfatés particulièrement intéressants comprennent l'héparine, la chondroïtine sulfate et le dextran sulfate ainsi que leurs dérivés synthétiques.

Les inventeurs ont également isolé l'ensemble du gène codant pour l'HBHA de l'ADN de M. *bovis* BCG. L'invention concerne donc une séquence nucléotidique caractérisée en ce qu'elle code pour une séquence peptidique permettant l'adhérence de mycobactéries à des cellules hôtes. Plus particulièrement, la séquence nucléotidique de l'invention code pour un antigène mycobactérien de type hémagglutinine de liaison à l'héparine (HBHA) notamment la partie C-terminale de la séquence peptidique de la figure 10.

L'invention concerne également un hôte cellulaire recombinant, caractérisé en ce qu'il comprend dans son génome une des séquences nucléotidiques décrites précédemment. Dans une des réalisations préférentielles de l'invention, l'hôte cellulaire recombinant mais non exclusif est le BCG pour lequel des vecteurs d'expression directement utilisables pour le développement de BCG recombinants utilisables chez l'homme ou l'animal ont été développés.

Le BCG est utilisé en thérapie, plus particulièrement en thérapie anti-cancéreuse et notamment contre les cancers superficiels de la vessie. Dans ce type d'application thérapeutique, il semblerait exister une corrélation entre la faculté d'adhérence du BCG et son pouvoir anti-tumoral. Dans le contexte de la présente invention, l'identification de la HBHA et le clonage de son gène rendent possible une augmentation de la capacité d'adhérence via une surexpression du gène codant la HBHA.

La description de la présente invention sera effectuée en se référant aux figures suivantes :
- la figure 1 représente l'effet de glucides sulfatés et non sulfatés sur l'adhérence mycobactérienne aux cellules CHO et aux macrophages;
- la figure 2 représente les données démontrant la purification d'une protéine de liaison d'héparine de M. *bovis* BCG;
- la figure 3 représente une comparaison de la protéine de liaison à l'héparine de M. *bovis* avec le complexe d'antigène 85;
- la figure 4 représente l'effet de glucides sulfatés et non sulfatés sur l'hémagglutination induite par HBHA;
- la figure 5 représente l'inhibition de l'adhérence de BCG aux cellules CHO par des anticorps anti-HBHA;
- la figure 6 représente des analyses par immunoempreintes effectuées avec des anti-sérums de tuberculeux;
- la figure 7 représente la séquence nucléotidique et la séquence d'acides aminés d'un fragment de HBHA déduite à partir d'un fragment PCR de l'ADN chromosomique de BCG;
- la figure 8 représente une analyse par Southern-blot de l'ADN chromosomique de BCG;
- la figure 9 représente la stratégie de séquençage du gène codant pour HBHA;
- la figure 10 représente la séquence d'ADN du gène de BCG codant pour HBHA;
- la figure 11 représente l'analyse par électrophorèse en gel polyacrylamide et par immunoblotting de l'expression de la HBHA chez *E. coli.*

### Description détaillée de l'invention

### Inhibition de l'adhérence mycobactérienne aux cellules épithéliales par des glucides sulfatés.

L'invention concerne l'utilisation de glucides sulfatés pour inhiber l'adhérence de mycobactéries à des cellules épithéliales. Pour démontrer que les mycobactéries peuvent adhérer par l'intermédiaire des glucides sulfatés, les inventeurs ont testé si des polysaccharides sulfatés solubles sont capables de réduire l'adhérence de *M*. *bovis* BCG aux cellules épithéliales.

Des M. *bovis* (BCG) (souche 1173P2, OMS, Stockholm, Suède, passages 3 à 8) en croissance exponentielle ont été marqués en cultivant les mycobactéries pendant trois jours dans du milieu de Sauton contenant 5 µCi/ml [6-³H]uracile (New England Nuclear, 24 Ci/mmole). Les mycobactéries ont ensuite été récoltées par centrifugation (3000 x g pendant 5 min), lavées deux fois par du tampon phosphate salin de Dulbecco (DPBS) et remises en suspension dans du milieu de culture RPMI 1640 contenant 300 mg/l L-glutamine (GIBCO), dépourvu de sérum de veau foetal (RPMI).

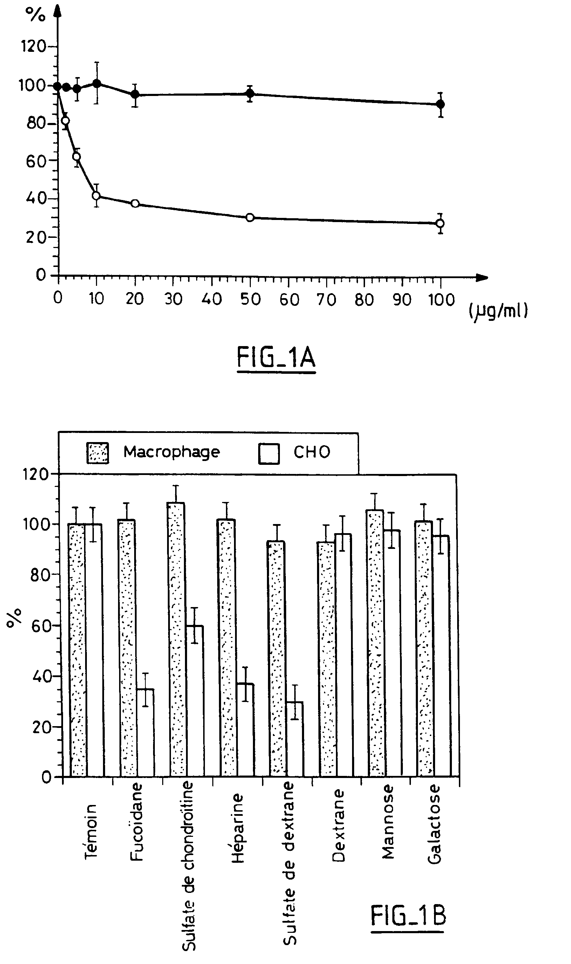

La veille de l'essai d'adhérence, les puits des plaques de culture de tissus à 24 puits (Nunclon, Nunc, Danemark) ont été inoculés avec 10⁵ cellules d'ovaires de hamsters Chinois (CHO) cultivées extemporanément (voir figures 1 A et 1 B, barres grises) ou des macrophages J774A.1 (ATCC TIB67) (voir figure 1B, barres noires) remis en suspension dans 2 ml de RPMI additionnés de 10 % (v/v) de sérum de veau foetal décomplémenté (RPMI-SVF). Juste avant l'essai, les cellules ont été lavées trois fois par 2 ml de RPMI, et on a ajouté à chaque puits 1 ml de suspension mycobactérienne dans du RPMI pour obtenir une multiplicité d'infection de 10 bactéries par cellule eucaryote.

L'essai d'adhérence a été réalisé en présence de concentrations croissantes de D(+)galactose (Sigma) (voir figure 1A, cercles noirs) ou d'héparine de la muqueuse intestinale porcine (Mᵣ 6 kDa, Sigma) (voir figure 1A, cercles blancs), ou avec 20 µg/ml des glucides indiqués (Sigma) (voir figure 1 B). Après 6 heures d'incubation à 37°C dans une atmosphère contenant 5 % de CO₂, les cellules ont été lavées trois fois par 2 ml de DPBS, et enfin lysées par l'addition de 1 ml d'eau distillée contenant 0,1 % (p/v) de déoxycholate de sodium. La radioactivité associée aux lysats cellulaires a été comptée en utilisant un compteur à scintillation liquide (Beckman, modèle LS 6000SC). L'adhérence résiduelle est exprimée en pourcentage de coups radioactifs par minute par rapport aux coups obtenus en l'absence du glucide. Les données des figures 1 A et 1 B représentent les moyennes d'expériences réalisées en quadruple, et les barres d'écart type sont représentées.

Comme le montre la fig. 1A. de faibles concentrations d'héparine ont inhibé sensiblement l'adhérence aux cellules CHO des BCG marqués par [³H]uracile, alors que le galactose jusqu'à 100 µg/ml n'a pas eu d'effet significatif. Du dextran sulfate, du fucoïdane et de la chondroïtine sulfate ont pu réduire l'adhérence, mais ont aucune inhibition significative n'a pu être observée avec du mannose ou du dextran non sulfaté, même aux plus fortes concentrations testées (1 mg/ml).

Ces résultats conduisent à proposer que les mycobactéries possèdent à leur surface une adhésine majeure de liaison des glucides sulfatés. Toutefois, comme l'inhibition de l'adhérence n'a pas dépassé 70 %, d'autres composants sont vraisemblablement impliqués dans ce processus. De façon intéressante, les interactions de BCG avec les macrophages J774A.1 n'ont pas été affectées par les glucides sulfatés, ni par les sucres non sulfatés (fig. 1 B), même à des concentrations allant jusqu'à 1 mg/ml (résultats non présentés). Ceci est en accord avec des rapports précédents mettant en cause les récepteurs au complément CR1, CR3 et CR4 comme ligands mycobactériens à la surface des monocytes sanguins et des macrophages alvéolaires (10-12).

### Purification d'une protéine de liaison à l'héparine présente chez M. bovis BCG et de M. tuberculosis.

L'invention concerne une séquence peptidique permettant l'adhérence de mycobactéries à des cellules hôtes. L'inhibition de l'adhérence de BCG aux cellules épithéliales par des sucres sulfatés suggérait déjà que les mycobactéries expriment une adhésine qui interagit avec des glycoconjugués sulfatés, tels des glycoprotéines ou glycolipides sulfatés, présents à la surface des cellules hôtes. Pour identifier les adhésines en question, des surnageants de culture de BCG ont été fractionnés par chromatographie sur héparine-Sépharose.

Des *M. bovis* BCG ont été cultivés à 37°C en cultures statiques dans des fioles de Roux de 175 cm² (Falcon, Becton-Dickinson) contenant approximativement 150 ml de milieu de Sauton. A la phase stationnaire, les cultures ont été centrifugées (10.000 x g pendant 20 minutes), et on a chargé 500 ml de surnageant sur une colonne (1 x 5 cm) d'héparine-Sépharose CL-6B (Pharmacia) équilibrée avec du DPBS. La colonne a ensuite été lavée par 100 ml de DPBS, et les molécules retenues ont été éluées par un gradient linéaire de NaCl 0-500 mM dans 100 ml de DPBS. Le débit pendant toutes les étapes a été maintenu à 1,5 ml/min., et on a suivi en continu l'absorbance à 280 nm de l'éluat qui fut récolté par fractions de 1 ml. Ces fractions furent analysées par SDS-PAGE (13) en utilisant un gel à 12 % suivi d'une coloration au Bleu Brillant de Coomassie R-250.

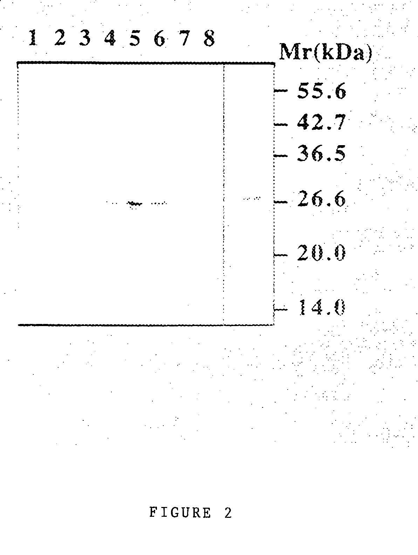

Les fractions analysées après le début du gradient sont montrées dans les pistes 1-8 de la figure 2. Les pistes de droite montrent la protéine de liaison à l'héparine purifiée à partir d'une préparation de parois cellulaires de *M. bovis* BCG. Les tailles des marqueurs de Mᵣ exprimées en kDa sont données dans la marge de droite.

Comme le montre la fig. 2, une protéine unique de 28 kDa est éluée à approximativement 350 mM de NaCl. L'interaction entre cette protéine et l'héparine est dépendante de la sulfatation du sucre car celle-ci pouvait également être purifiée en utilisant des billes de dextran sulfate, mais pas des billes de dextran (non présenté). La même protéine de liaison à l'héparine a également été purifiée à partir des filtrats de culture de M. *tuberculosis.*

L'obtention de deux protéines de liaison à l'héparine semblables à partir de deux souches mycobactériennes différentes confirme que des protéines de ce type sont présentes dans la plupart des souches mycobactériennes pathogènes. Des pourcentages d'homologie inférieurs à 100% peuvent conduire à des différences structurales, bien que les propriétés fondamentales de ces protéines n'en soient pas affectées. L'invention concerne donc également les protéines de liaison à l'héparine ayant une structure apparentée à la protéine isolée et pouvant être obtenues à partir de leurs propriétés d'adhérence à des glucides sulfatés. Ces protéines peuvent se distinguer de la protéine de l'invention par des additions, substitutions et délétions d'acides aminés sans que cela n'affecte de façon substantielle leurs propriétés d'adhérence aux cellules épithéliales.

### Localisation à la surface cellulaire de la protéine mycobactérienne de liaison à l'héparine.

Pour déterminer si cette protéine est une protéine strictement sécrétée ou si elle peut être associée à la surface mycobactérienne, des fractions de parois cellulaires ont été préparées à partir de BCG, puis soumises à une chromatographie sur héparine-Sépharose.

Pour les préparations de parois cellulaires, les mycobactéries ont été cultivées dans 2 litres de milieu de Sauton ou encore de milieu synthétique de Long (Quality Biological Inc., Gaithersburg, MD) pendant 12 à 14 jours. Les bactéries ont ensuite été récoltées par centrifugation, lavées une fois dans du DPBS contenant 0,05 % (v/v) de Tween 80 (DPBS/Tw), remises en suspension dans 100 ml de DPBS/Tw et chauffées à 80°C pendant 1 heure. Les bactéries ont été centrifugées à 13.000 x g pendant 20 min., lavées par du DPBS/Tw, remises en suspension dans 25 ml de DPBS/Tw contenant 5 mM d'inhibiteur de protéase AEBSF, RNase A et DNase I, soniquées de façon intermittente pendant 25 min., puis centrifugées à 13.000 x g pendant 20 min. Cette étape a été répétée, et les surnageants ont été réunis et centrifugés à 34.000 x g pendant 3 heures à 4°C. Le culot a été jeté et le surnageant final dilué 1:2 dans du DPBS et chromatographié sur héparine-Sépharose CL-6B.

Comme le montre la fig. 2, la protéine de liaison à l'héparine de 28 kDa était également présente dans ces préparations, ce qui confirme qu'elle est associée à la surface. Elle était également présente dans des fractions de parois cellulaires de *M. tuberculosis* et des préparations de tuberculine (dérivé protéique purifié antigène pour cutiréaction) dérivées de *M. tuberculosis.*

### Activité d'hémagglutination de la protéine de liaison à l'héparine mycobactérienne.

La capacité des adhésines bactériennes à agglutiner les globules rouges est souvent utilisée comme modèle pour étudier la fixation microbienne à la manière des lectines au récepteur des cellules eucaryotes. Les inventeurs ont par conséquent testé la protéine de liaison à l'héparine purifiée pour sa capacité à agglutiner des erythrocytes.

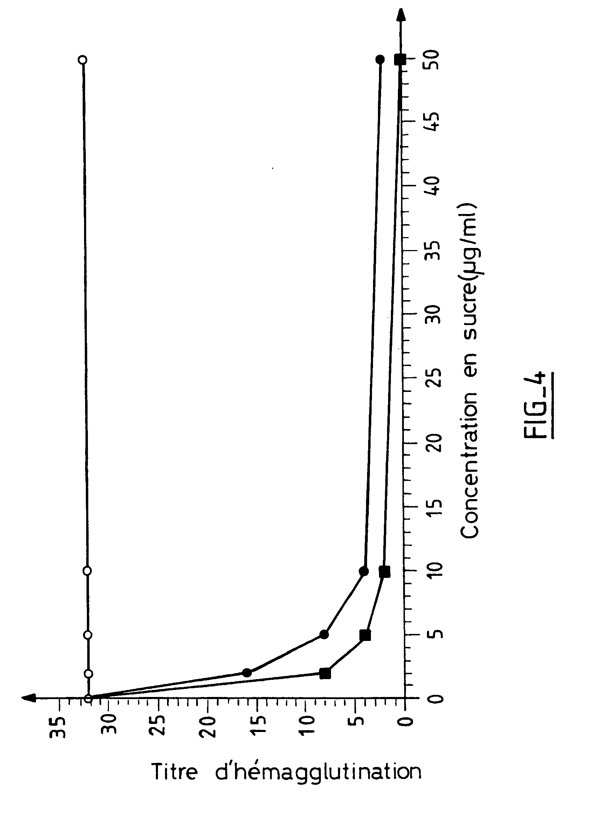

L'activité d'hémagglutination de la protéine de liaison à l'héparine purifiée de surnageants de culture a été dosée dans des plaques de microtitration en U (Falcon) en l'absence ou en présence d'héparine (figure 4, carrés noirs) de la muqueuse intestinale porcine, de dextran sulfate (figure 4, cercles noirs) ou de dextran (figure 4, cercles blancs) à des concentrations allant de 0 à 50 µg/ml. Des essais standard contenaient 70 µl d'une suspension fraîche d'érythrocytes de lapin préparée dans du DPBS et 70 µl d'HBHA purifiée correspondant à 1 µg de protéine. Les titres d'hémagglutination ont été lus après 5 heures d'incubation à température ambiante. Les données représentent des moyennes pour des expériences en quadruple, et les barres d'écart types sont montrées.

Moins de 0,1 µg de protéine purifiée a pu induire l'hémagglutination d'érythrocytes de lapin, mais pas d'érythrocytes d'humain, de mouton, d'oie ou de poulet (données non présentées). C'est pour cette raison que les inventeurs ont désigné cette protéine comme étant une hémagglutinine de liaison à l'héparine (HBHA). L'hémagglutination induite par HBHA a été inhibée par l'héparine ou le dextran sulfate, mais pas par le dextran (voir fig. 4), ce qui est similaire aux résultats obtenus dans l'essai d'adhérence des mycobactéries aux cellules CHO. L'activité d'hémagglutination est sensible à la chaleur dans la mesure où l'incubation de la protéine pendant 60 min à 80°C a aboli l'hémagglutination.

### Inhibition des activités d'hémagglutination et d'adhérence provenant de la médiation par HBHA, par des anticorps polyclonaux et monoclonaux spécifiques.

Dans la mesure où d'une part l'adhérence mycobactérienne aux cellules épithéliales est inhibée par des glucides sulfatés, et d'autre part une protéine de 28 kDa était purifiée par chromatographie sur héparine-Sépharose, il était important d'établir si l'adhérence mycobactérienne provenait de la médiation par HBHA.

Par conséquent, des anticorps polyclonaux de rat anti-HBHA ont été préparés de la manière suivante: 200 µg d'HBHA de BCG purifiée ont été soumis à l'électrophorèse préparative sur un gel de polyacrylamide à 15 % en présence de SDS, puis électro-transférés sur une membrane de nitrocellulose. Après une coloration rapide au rouge ponceau, les bandes correspondantes à l'HBHA ont été excisées avec précaution, découpées en petits carrés et soniquées brièvement dans 1,5 ml de PBS stérile. Après l'addition de 1 ml d'une solution de monophosphoryle-lipide A (MPL + Système Adjuvant TDM, Sigma Chemical Co., St. Louis, MO) préparée selon la recommandation du fabricant, deux rats Fischer ont été immunisés chacun avec 1 ml de la suspension d'antigène. Pour chaque immunisation, on a administré 400 µl par voie intrapéritonéale et deux fois 300 µl par voie sous-cutanée. Les animaux ont reçu des rappels de la même manière avec la même quantité d'antigène un mois plus tard, et le sérum a été récupéré 3 semaines après le rappel.

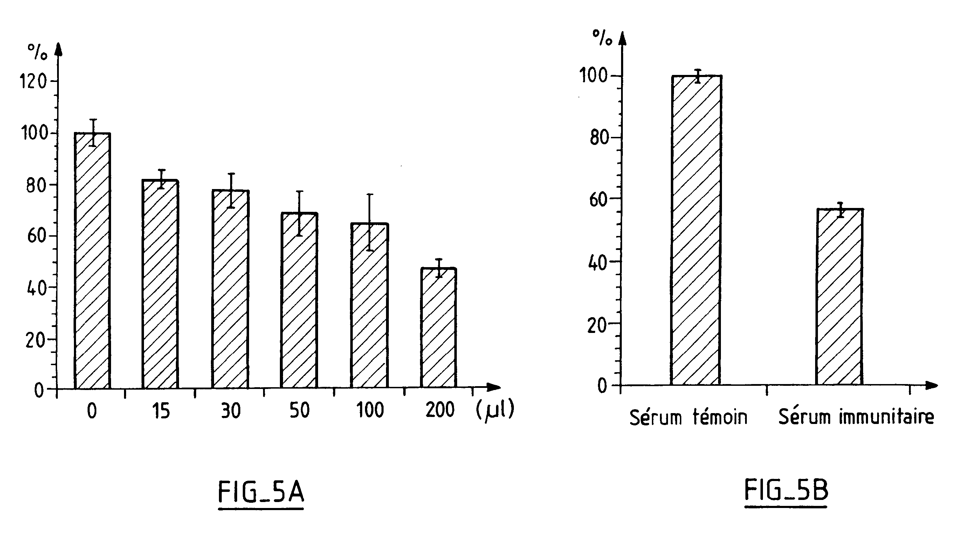

Le BCG a été marqué par [6-³H]uracile comme décrit ci-dessus. Approximativement 4 · 10⁶ BCG radiomarqués ont été remis en suspension dans 1 ml de PBS et préincubés avec les volumes indiqués de liquides ascétiques d'anticorps monoclonal 3921 E4 (14) anti-HBHA (figure 5, panneau A), ou avec 250 µl d'antisérum polyclonal de rat anti-HBHA (sérum immunitaire, figure 5, panneau B) ou de sérum naïf (figure 5, sérum témoin, panneau B) pendant 30 min. à température ambiante. Les suspensions bactériennes ont ensuite été lavées deux fois par 2 ml de DPBS pour éliminer les anticorps non liés, puis utilisées dans l'essai d'adhérence de cellules CHO à une multiplicité d'infection de 10, comme décrit précédemment.

Comme le montre la fig. 5, l'adhérence de BCG aux cellules CHO a été sensiblement inhibée d'une manière dose-dépendante par la présence d'anticorps anti-HBHA. Les liquides d'ascite contenant des anticorps monoclonaux non pertinents ou des antisérums naïfs n'ont eu aucun effet. Ces observations indiquent que l'adhérence des mycobactéries aux cellules épithéliales provient en partie de la médiation par HBHA.

### Etude des caractéristiques de HBHA.

Comme la taille de cette protéine est proche de celle des protéines de liaison de fibronectine du complexe d'antigène 85 (15), des analyses Western-blot ont été utilisées pour déterminer si elles étaient apparentées.

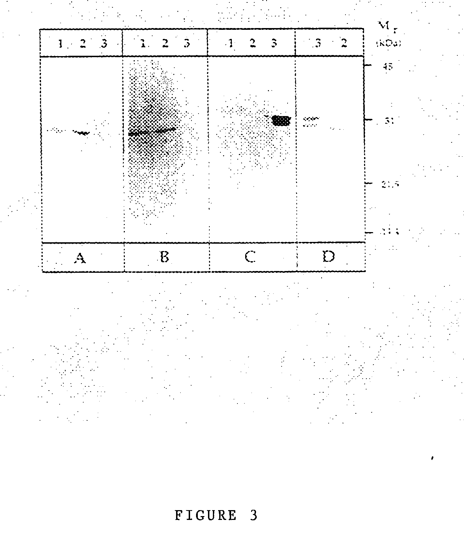

La protéine de liaison à l'héparine purifiée d'une préparation de paroi cellulaire de *M. bovis* BCG (figure 3, pistes 1) ou de surnageant de culture (pistes 2) a été comparée avec le complexe d'antigène 85 purifié (pistes 3) par analyse d'immunoempreinte (panneaux A, B, C) et coloration au Bleu de Coomassie (panneau D) après SDS-PAGE. L'analyse par immunoempreinte a été effectuée en utilisant des anticorps polyclonaux dressés contre la protéine de liaison à l'héparine purifiée (panneau A), l'anticorps monoclonal 4057D2 (panneau B), ou les anticorps polyclonaux dressés contre le complexe d'antigène 85 (panneau C). Les pistes 1 et 2 des panneaux A, B, et C contiennent 2 µg de protéine purifiée, les pistes 3 des panneaux A, B et C contiennent 7 µg de protéine purifiée, les pistes 2 et 3 du panneau D contiennent 4 µg et 15 µg de protéine purifiée respectivement. Les tailles des marqueurs de Mᵣ sont données dans la marge.

La fig. 3 montre que des anticorps polyclonaux dressés contre la protéine de liaison à l'héparine de 28 kDa purifiée de BCG n'ont pas reconnu les protéines du complexe d'antigène 85 purifiées. A l'inverse, des anticorps polyclonaux et monoclonaux (non présentés) dressés contre le complexe d'antigène 85 de BCG n'ont pas réussi à reconnaître la protéine de liaison à l'héparine, ce qui implique qu'il s'agit de protéines distinctes. Ce résultat est également appuyé par les différents profils de migration de ces protéines pendant la SDS-PAGE (fig. 3, panneau D).

Les protéines de liaison à l'héparine purifiées de *M.tuberculosis* H37Ra et de BCG ont été soumises à un séquençage des acides aminés N-terminaux. Pour ce faire, 25 µg d'HBHA ont été soumis à l'électrophorèse sur gel de polyacrylamide-SDS en utilisant un gel de polyacrylamide à 15 %. Après l'électrophorèse, la matière a été transférée sur une membrane PVDF (ProBlott, ABI) par électroempreinte. Après coloration par le bleu de Coomassie, la bande correspondant à l'HBHA a été excisée et soumise à une dégradation d'Edman automatisée. Les 16 premiers acides aminés étaient Ala-Glu-Asn-Ser-Asn-Ile-Asp-Asp-Ile-Lys-Ala-Pro-Leu-Leu-Ala-Ala. Les 16 premiers acides aminés de la protéine de liaison à l'héparine de BCG ont également été déterminés et se sont avérés identiques à ceux de *M. tuberculosis.* Une recherche de similarité dans les bases de données des protéines a mis en évidence que la protéine de liaison à l'héparine n'avait pas été identifiée auparavant, et qu'elle représente par conséquent une nouvelle protéine mycobactérienne. En outre, les 16 premiers acides aminés n'ont présenté aucune similarité de séquence significative par rapport à d'autres séquences de protéines connues.

### Clonage du gène de BCG codant pour HBHA.

Pour le clonage du gène codant pour HBHA, on a d'abord déterminé les séquences N-terminales des fragments internes de HBHA. Pour cela, de l'HBHA purifiée a été soumise à l'électrophorèse comme décrit précédemment. Après l'électrophorèse, la protéine a été digérée par la trypsine à l'intérieur du gel. Les peptides résultants ont alors été isolés par HPLC en phase inverse, puis soumis à la dégradation d'Edman. Quatre peptides ont permis la détermination de séquence:
Le peptide S 1441: Lys-Ala-Glu-Gly-Tyr-Leu-Glu-Ala-Ala-Thr
Le peptide S1443: Xxx-Glu-Gly-Tyr-Val-Asp-Gln-Ala-Val-Glu-Leu-Thr-Gln-Glu-Ala-Leu-Gly-Lys
Le peptide S 1446: Xxx-Gln-Glu-Xxx-Leu-Pro-Glu-Xxx-Leu
Le peptide S 1447: Phe-Thr-Ala-Glu-Glu-Leu-Arg.

La séquence de deux paires d'oligonucléotides était dérivée des séquences peptidiques internes de HBHA. La teneur en G + C généralement forte de l'ADN mycobactérien a conduit les inventeurs à favoriser G ou C dans la troisième position des codons ("wobble"). Le premier couple d'oligonucléotides provenait du peptide S1441 et avait les séquences suivantes: 5'AAG GC(G/C) GAG GG(G/C) TAC CT 3' (oligo S1441) et 5' AGG TA(G/C) CCC TC(G/C) GCC TT 3' (oligo S1441 inverse). Le deuxième couple d'oligonucléotides provenait du peptide S1443 et avait les séquences suivantes: 5'GAC CAG GC(G/C) GT(G/C) GAG CT 3' (oligo S1443) et 5' AGC TC(G/C) AC(G/C) GCC TGG TC 3' (oligo S1443 inverse).

L'ADN chromosomique de BCG a été extrait comme décrit par Kremer et al. (16). Les réactions de polymérisation en chaîne (PCR) en utilisant 50 ng d'ADN chromosomique de BCG et 1 µg soit des oligo S1441 et S1443 inverse, soit des oligo S1443 et S1441 inverse ont été effectuées à une température d'hybridation de 50°C et à un nombre de cycles de PCR de 30. Seule la réaction par PCR effectuée avec les oligonucléotides S1441 et S1443 inverse a donné un fragment d'ADN amplifié spécifique d'approximativement 150 bp. Ce fragment amplifié était encore observé lorsque la température d'hybridation a été augmentée à 57°C.

Le fragment amplifié a été inséré dans le site *Hind*ll de pUC18 (Boehringer, Mannheim) et introduit dans *Escherichia coli* XL1-Blue (New England Biolabs). La teneur en plasmide de *E*. *coli* recombinant a ensuite été analysée par des méthodes standard (17), et le plasmide contenant le fragment attendu a été appelé pClone5.

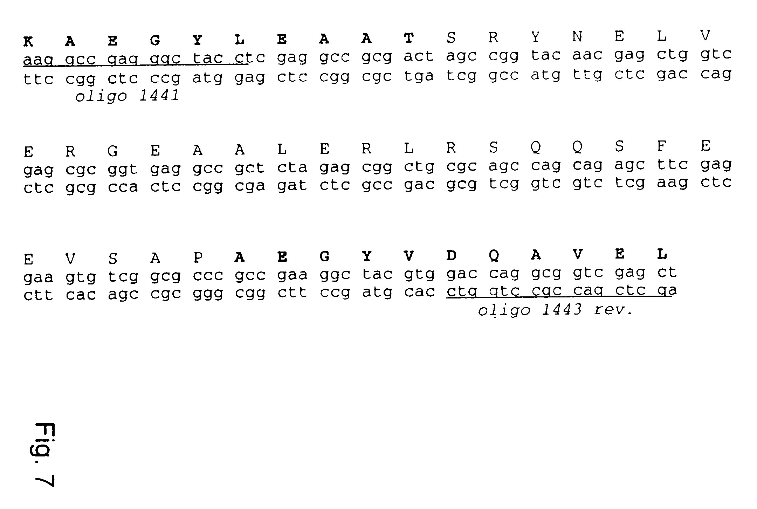

Après purification par chromatographie sur une colonne de Nucleobond AX (Macherey - Nagel) selon les instructions du fournisseur, un fragment d'ADN bicaténaire d'approximativement 150 pb a été séquencé par la méthode de terminaison d'élongation de chaîne didésoxyribonuclétidique en utilisant du [alpha-³⁵S]dCTP (1,000 Ci/mmole; Amersham) et le kit de séquençage d'ADN de T7 (Pharmacia) selon les instructions du fabricant. La séquence obtenue est présentée à la fig. 7 dans laquelle la séquence des deux oligonucléotides utilisées pour la PCR est soulignée. La séquence d'acides aminés déduite de la séquence d'ADN s'est avérée correspondre aux séquences d'acides aminés déterminées pour les peptides S1441 et S1443. Ceci indiquait que le fragment PCR amplifié correspond à une partie interne du gène de BCG codant pour HBHA.

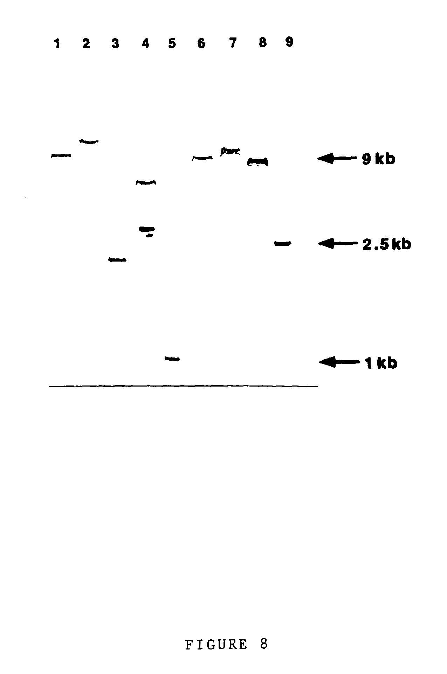

Pour cloner l'ensemble du gène codant pour HBHA, le fragment de 150 pb de pClone5 a été excisé par digestion par les enzymes de restriction *Bam*HI et *Hind*III. Le fragment a ensuite été purifié par électrophorèse sur un gel de polyacrylamide à 7 % et excisé du gel par électro-élution. Le fragment purifié a été marqué à la digoxigénine en utilisant le kit de détection et de marquage d'ADN par DIG (Boehringer) comme recommandé par le fabricant. Le fragment marqué a ensuite été utilisé dans des expériences de Southern-blot pour sonder l'ADN chromosomique de BCG digéré par *Bam*Hl (figure 8, voie 1), *Eco*Rl (figure 8, voie 2), *Pst*l (figure 8, voie 3), *Sma*l (figure 8, voie 4), *Acc*l (figure 8, voie 5), *Nco*l (figure 8, voie 6), *Not*l (figure 8, voie 7), *Sac*l (figure 8, voie 8) ou *Sph*l (figure 8, voie 9), soumis à une électrophorèse sur agarose et transféré sur une membrane de nylon. La membrane a ensuite été sondée avec le fragment BamHI/HindII d'approximativement 150 pb de pClone5. Les tailles de marqueurs sont montrées dans la marge de droite. Des analyses de Southern-blot ont été effectuées en utilisant des protocoles standard (17). Comme le montre la fig. 8, la digestion par *Sph*I de l'ADN chromosomique de BCG a donné un fragment unique d'environ 2,5 kb qui s'hybridait avec la sonde.

Les fragments de restriction *Sph*I de l'ADN chromosomique de BCG de 2,3 à 2,7 kb ont alors été purifiés par électrophorèse préparative et électro-élution, et insérés dans le site *Sph*I de pUC18. Les plasmides recombinants ont été utilisés pour transformer *E*. *coli* XL1-Blue. Des colonies blanches cultivées sur gélose LB (17) additionnées d'ampicilline (150 µg/ml) d'iso-propyl-thiogalactopyrannoside (IPTG, 40 µg/ml) et de X-gal (40 µg/ml) ont été analysées par hybridation sur colonies en utilisant la sonde marquée à la digoxigénine. Parmi approximativement 300 colonies analysées, une s'est hybridée avec la sonde. L'analyse par restriction du plasmide isolé de ces clones a indiqué qu'il contenait un fragment Sphl de 2,5 kb qui s'est hybridé avec la sonde en Southern-blot.

### Analyse de la séquence du gène de BCG codant pour HBHA.

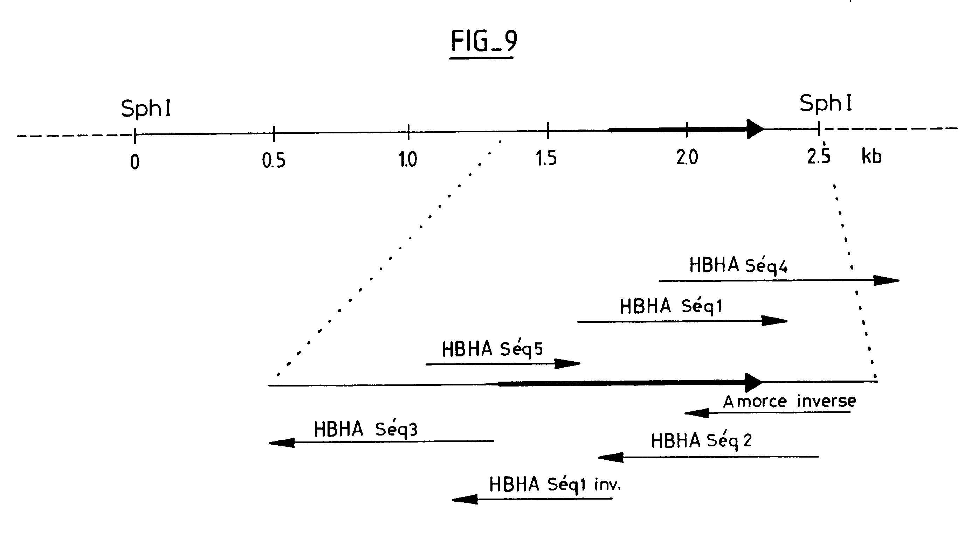

Le gène codant pour HBHA contenu dans le fragment Sphl de 2,5 kb a été séquencé en utilisant la méthode de terminaison d'élongation de chaîne didésoxyribonucléotidique décrite ci-dessus. Les séquences des oligonucléotides synthétiques utilisés pour le séquençage sont présentées au tableau 1, et la stratégie de séquençage est représentée à la fig. 9. Le fragment *Sph*I de 2,5 kb cloné est représenté par le trait noir. Les pointillés représentent l'ADN vecteur. Les flèches épaisses représentent le cadre ouvert de lecture d'HBHA. Les flèches fines indiquent la direction et la longueur du fragment d'ADN séquencé pour chaque oligonucléotide indiqué au-dessus de sa flèche respective.

**TABLEAU I: Oligonucléotides utilisés pour le séquençage du gène codant pour HBHA.**

| Nom de l'oligonucléotide | séquence |
|---|---|
| HBHA Seq1 | 5'AGC CGG TAC AAC GAG CTG GTC 3' |
| HBHA Seq1 Inv | 5'GAC CAG CTC GTT GTA CCG GCT 3' |
| HBHA Seq2 | 5' CAT CCA ACA CGT CGA CTC C 3' |
| HBHA Seq3 | 5' TTG ATG TCA TCA ATG TTC G 3' |
| HBHA Seq4 | 5' CGT GGA CCA GGC GGT GGA G 3' |
| HBHA Seq5 | 5' GAC GAT CAG GAG GTT TCC CCG 3' |
| Amorce inverse | 5' AGC GGA TAA CAA TTT CAC ACA GGA 3' |

L'ensemble du gène codant pour HBHA est localisé à l'intérieur du fragment S*ph*l de 2,5 kb, à une extrémité du fragment, et il a été totalement séquencé à partir des deux brins.

La séquence nucléotidique ainsi que la séquence protéique dérivée est présentée à la fig. 10. Le cadre ouvert de lecture a été mis en évidence entre les nucléotides 331 et 927. Les 16 premiers codons après l'éventuel codon d'initiation ATG 331/333 se sont traduits en une séquence d'acides aminés identique à celle déterminée après séquençage N-terminale de la protéine HPHA purifiée. En amont de l'éventuel codon d'initiation, aux résidus 331-333, on a trouvé un codon d'arrêt TAG en phase aux positions 310-312. Ceci suggère fortement que le cadre ouvert de lecture d'HBHA ne contient pas de séquences codant pour le peptide signal classique, et 331/333 représente donc probablement le codon d'initiation. Ceci est soutenue par la présence d'un site putatif de liaison des ribosomes AGGAA que l'on trouve de 10 à 6 nucléotides en amont du codon d'initiation.

La séquence protéique déduite présente un poids moléculaire calculé de21390, ce qui est inférieur au poids moléculaire apparent estimé par SDS-PAGE. La protéine prédite est composée de 198 acides aminés et ne contient pas de cystéine, de méthionine, d'histidine ou de tryptophane. Les cinq séquences peptidiques déterminées ont été retrouvées dans la séquence protéique prédite de HBHA (souligné à la fig. 10). Des recherches d'homologie dans les bases de données de protéines ont confirmé que HBHA est une protéine mycobactérienne non identifiée jusqu'ici.

Les inventeurs ont identifié une région de la protéine susceptible d'être impliquée dans les interactions avec les glycoconjugués sulfatés. Cette région est comprise dans la séquence suivante :

KKAAPAKKAAPAKKAAPAKKAAAKKAPAKKAAAKKVTQK.

Des peptides particulièrement susceptibles d'être impliqués dans les interactions sont les suivants :
KKAAPA
KKAAAKK.

Les répétitions à l'extrémité C-terminale de la protéine, riches en prolines, alanines et lysines, pourraient expliquer la différence entre les poids moléculaires apparents et calculés de la protéine qui subit des modifications post-traductionnelles telles que la glycosylation.

L'invention concerne donc plus particulièrement la portion C-terminale de la séquence de la figure 10 et plus particulièrement la séquence comprenant approximativement les 50 derniers acides aminés entre les acides aminés 150 et 199 de la séquence de la figure 8 10. De façon particulièrement préférée, l'invention concerne l'extrémité C-terminale de la protéine de la figure 10, et plus particulièrement l'extrémité C-terminale comprenant les répétitions riches en proline, alanine et lysine, soit les 50 derniers acides aminés de la séquence de la figure 10.

On peut également envisager tout variant des séquences décrites ci-dessus obtenu par addition, substitution ou délétion d'un ou plusieurs acides aminés sans modifier de manière substantielle les propriétés de la région d'intérêt. Parmi les modifications envisagées, on note tout particulièrement les mutations silencieuses au niveau des séquences nucléotidiques qui ne modifient pas la séquence peptidique de la protéine ou du fragment d'intérêt ainsi que les mutations conservatives qui consistent à substituer un ou plusieurs acides aminés présentant les mêmes caractéristiques fonctionnelles que l'acide aminé de la séquence native. Des additions ou délétions d'acides aminés sans que les propriétés des régions d'intérêt soient substantiellement modifiées font également partie de l'invention. Certains des résidus peuvent être éliminés ou modifiés en exprimant un gène modifié de cette manière chez *E*. *coli* comme décrit pour le gène complet. Si cette protéine fixe l'héparine avec la même affinité que la HBHA entière, cela signifie que les séquences répétées ne sont pas impliquées dans cette fixation. En revanche, si la protéine modifiée perd la capacité d'interagir avec l'héparine, la région modifiée de la protéine joue un rôle dans cette interaction.

### Expression du gène de la HBHA de BCG chez Escherichia coli

L'invention concerne également un hôte cellulaire recombinant, caractérisé en ce qu'il comprend dans son génome une des séquences nucléotidiques décrites précédemment. Plus particulièrement, l'invention concerne la transformation d'hôtes cellulaires tels que *E. coli* avec une des séquences nucléotidiques de l'invention afin de produire l'antigène mycobactérien HBHA permettant l'adhérence des mycobactéries aux glucides sulfatés des cellules épithéliales. La souche bactérienne à transformer peut comprendre la séquence nucléotidique codant pour la portion C-terminale du polypeptide HBHA de la figure 10, codant pour les 50 derniers acides aminés du polypeptide HBHA ou codant pour les 30 à 50 derniers acides aminés du polypeptide HBHA.

Afin de produire la HBHA de BCG dans *Escherichia coli,* le plasmide dérivé de pUC18 qui contient le fragment Sphl de 2.5 kb comportant le gène complet de la HBHA de BCG fut restreint simultanément par *Nco* l et *Kpn* l. Le fragment de 705 paires de bases issu de cette double restriction fut purifié par électroélution après migration dans un gel d'agarose de 1% selon des procédures standards (17) et finalement cloné dans le vecteur d'expression pKK388-1 (Clontech, Palo Alto, Ca., UA) préalablement restreint par *Nco* l et *Kpn* l. Le plasmide recombinant fut ensuite introduit dans *E*. *coli* XL1-Blue par des techniques classiques de transformation (17).

L'analyse phénotypique de la souche portant ce plasmide d'expression fut réalisée en cultivant celle-ci dans un milieu LB liquide et en stimulant la production de la HBHA recombinante par l'ajout d'isopropylthiogalactopyranoside (IPTG) selon des méthodes standards (17). Pratiquement, deux cultures en erlenmeyers de 500 ml contenant chacun 100 ml de milieu LB liquide supplémenté avec de l'ampicilline à raison de 150 µg/ml, furent respectivement inoculées avec 4 ml de préculture *E. coli* XL1-Blue contenant le plasmide d'expression de la HBHA ou le plasmide pKK388-1 représentant la culture contrôle. La croissance des deux cultures fut suivie par mesure de la densité optique à 600 nm. Quand celle-ci fut de 0,6, de l'IPTG fut ajouté aux cultures à raison de 1 mM final et les cultures furent prolongées pendant 4 heures. Des échantillons de culture furent prélevées avant l'ajout d'IPTG et au terme des 4 heures de culture en présence de l'inducteur afin d'analyser par électrophorèse en gel de polyacrylamide et par immunoblotting la production de HBHA recombinante.

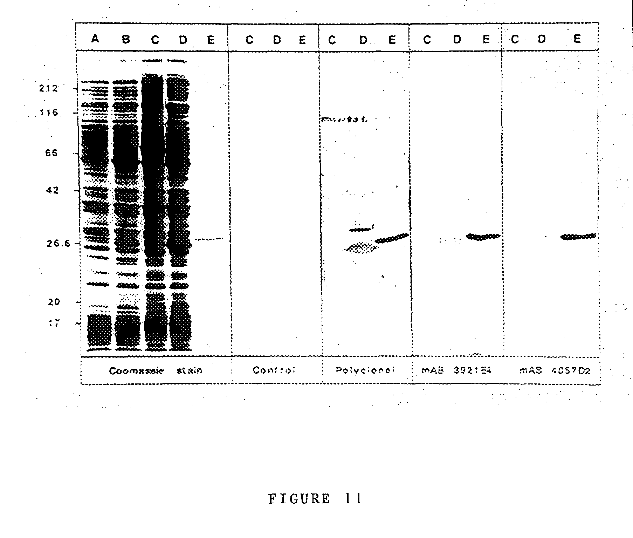

Les résultats de ces différentes analyses sont repris à la figure 11.

Les pistes A et B représentent des lysats totaux avant induction de *E*. *coli* XL1-Blue transformée respectivement par pKK388-1 et le dérivé de pKK388-1 contenant le gène codant la HBHA. Les pistes C et D représentent des lysats totaux après induction à l'IPTG de *E*. *coli* XL1-Blue transformée respectivement par pKK388-1 et le dérivé de pKK388-1 contenant le gène codant la HBHA. Les pistes E contiennent 1 µg de HBHA purifiée à partir de parois de BCG. De gauche à droite, les différents panneaux représentent respectivement la coloration au bleu de Coomassie R-250 du gel de polyacrylamide et les immunoblots sondés par un sérum murin contrôle, un sérum murin dirigé contre la HBHA de BCG, l'anticorps monoclonal murin 3921 E4 et l'anticorps monoclonal murin 4057D2. Les poids moléculaires de référence sont inscrits à la gauche du panneau présentant la coloration au bleu de Coomassie du gel de polyacrylamide.

L'analyse en gel de polyacrylamide coloré au bleu de Coomassie montre la synthèse d'un polypeptide d'environ 27 kDa chez *E*. *coli* XL1-Blue portant le vecteur d'expression de la HBHA et déréprimé par l'IPTG. Ce polypeptide reconnu en immunoblotting par un antisérum murin dirigé contre la protéine HBHA purifié d'un surnageant de culture de BCG n'est pas produit par *E. coli* XL1-Blue portant le vecteur pKK388-1 sans insert, montrant que sa synthèse dépend de la séquence d'ADN de BCG cloné dans le vecteur d'expression. Comme celle-ci est intégralement la séquence codante de la HBHA de BCG, il fut surprenant d'observer que le polypeptide recombinant présente un poids moléculaire apparent plus faible que celui de la HBHA purifiée de BCG et qui est de 28 kDa dans le système de gel considéré.

L'invention concerne donc également une séquence peptidique recombinante caractérisée en ce qu'elle permet l'adhérence de mycobactéries à des cellules hôtes. Plus particulièrement, l'invention concerne une séquence peptidique comprenant un polypeptide d'environ 27 kDa reconnu par l'anticorps monoclonal 3921 E4 (14) et non reconnu par l'anticorps monoclonal 4057D2 (14). De façon préférée, la séquence peptidique recombinante de l'invention est le produit d'expression d'une souche *E. coli* transformée avec une des séquences nucléotidiques décrites précédemment et codant pour une séquence peptidique permettant l'adhérence de mycobactéries à des cellules hôtes, plus particulièrement une séquence nucléotidique obtenue à partir de *M. bovis* BCG ou M. *tuberculosis.*

Comme le poids moléculaire de la HBHA déduit à partir de la séquence de son gène est significativement inférieur à son poids moléculaire apparent observé après migration électrophorétique et que cette différence est le résultat de modifications post-traductionnelles de la HBHA, ces modifications ne sont pas réalisées dans *E. coli.* Cette conclusion est étayée par le fait que nous observons une immuno-réactivité différentielle de la HBHA recombinante avec les deux anticorps monoclonaux 3921 E4 et 4057D2. Si ces deux anticorps monoclonaux reconnaissent de manière équivalente en immunoblotting la HBHA purifiée de BCG, seulement 3921 E4 est immunoréactif avec la HBHA recombinante produite chez E. coli. Cette observation montre tout d'abord que les épitopes de ces deux anticorps monoclonaux sont différents et que l'épitope reconnu par 4057D2 sur la HBHA n'est plus présent quand celle-ci est produite chez *E*. *coli,* qu'il est localisé sur un élément moléculaire résultant d'une modification post-traductionnelle non réalisée par *E*. *coli.*

Cette modification post-traductionnelle est une glycosylation dans la mesure où il a déjà été montré que la partie saccharidique d'une glycoprotéine pouvait être immunogénique. Le motif saccharidique est très immunogénique dans la mesure où l'analyse par immunoblotting utilisant le sérum murin anti-HBHA donne lieu à un plus faible signal avec la HBHA recombinante par rapport à celui observé avec la HBHA purifié de BCG. Cette observation est d'autant plus interpellante que la quantité de HBHA recombinante mise en jeu dans cette analyse est largement supérieure à celle de la HBHA naturelle, comme l'atteste la coloration au bleu de Coomassie du gel de polyacrylamide. Par ailleurs, comme l'immunoréactivité de 3921 E4 est proche de celle observée avec le sérum murin, l'épitope reconnu par cet anticorps monoclonal est donc partiellement dépendant de la modification post-traductionnelle suspectée.

Plusieurs approches expérimentales ont été envisagés pour élucider la nature exacte de cette modification post-traductionnelle. Tout d'abord, la HBHA purifiée à partir de BCG est soumise à l'action de différents endoglycosidases ou exoglycosidases afin de voir si le poids moléculaire apparent de cette protéine diminue après action de ces enzymes. Une plus rapide migration électrophorétique observée après digestion avec une ou plusieurs de ces enzymes, renseigne directement sur la nature chimique de la modification saccharidique. Parallèlement, des cultures de BCG menées en présence d'inhibiteurs de glycosylation (ex. tunicamycine qui inhibe la N-glycosylation chez les bactéries gram-positives) constituent une approche intéressante pour élucider la nature des modifications post-traductionnelles de la HBHA.

### Identification du site de fixation à l'héparine (« Heparin-binding site ») de la HBHA.

Comme il a été démontré que la HBHA a un rôle direct dans l'interaction entre le BCG et les cellules épithéliales, interaction qui est inhibable par l'héparine, il est important d'identifier la région qui est impliquée dans l'interaction du BCG avec les cellules épithéliales via les polysaccharides sulfatés.

La HBHA produite sous forme recombinante par E. coli est l'objet d'une protéolyse affectant son extrémité carboxyterminale et entraînant une diminution d'affinité pour l'héparine

La HBHA produite chez E. coli WL 1 Blue s'accumule dans le cytoplasme mais demeure soluble puisqu'une sonication des colibacilles suivie d'une centrifugation de clarification (10.000 g pendant 15 minutes) permet de retrouver l'intégralité de la HBHA détectable en immunoblot dans la fraction soluble du lysat. Lorsque cette dernière est incubée à température ambiante, la HBHA recombinante subit une dégradation rapide jusqu'au terme d'une incubation de 2 heures, son poids moléculaire apparent passe de 27 kDa à 19 kDa. La forme de 19 kDa qui n'est plus reconnue par l'anticorps monoclonal 3921 E4 montre cependant une bonne résistance à une dégradation ultérieure puisque cette forme demeure stable au terme d'une incubation de 18 heures à température ambiante. Comme la dégradation de la HBHA recombinante a pu être stoppée par ajout d'1mM d'AEBSF qui est un inhibiteur de protéases à sérine, il est probable que cette dégradation soit le résultat d'une protéolyse. Il est à noter que de la HBHA native incubée dans un lysat de E. coli XL1-Blue ne subit pas pareille dégradation, suggérant ainsi que la modification post-traductionnelle portée par la HBHA native lui confère une résistance vis-à-vis de la protéolyse observée avec la HBHA recombinante.

L'analyse chromatographique sur héparine-Sépharose d'un sonicat clarifié de E. coli XL1-Blue produisant de la HBHA recombinante et supplémenté avec 1 mM d'AEBSF, a révélé que la HBHA recombinante fixe l'héparine avec la même affinité que la HBHA native puisqu'elle élue à 350mM NaCl in PBS. Par contre, la HBHA dégradée en polypeptide de 19 kDa ne fixe plus l'héparine ce qui montre que la partie clivée est indispensable pour la reconnaissance des polysaccharides sulfatés. La HBHA recombinante purifiée sur héparine-Sépharose s'est en outre révélée incapable d'agglutiner les érythrocytes de lapin. Ces observations montrent que la modification post-traductionnelles de la HBHA n'est pas impliquée dans son activité de fixation à l'héparine mais est importante pour son activité d'hémagglutination.

L'analyse de la HBHA recombinante par chromatographie sur matrice d'héparine immobilisée s'est aussi révélée intéressante pour la cartographie du domaine protéique impliqué dans la reconnaissance de l'héparine (Heparin-binding site). En effet, trois formes de HBHA recombinante présentant des poids moléculaires de 27, 26 et 25 kDa furent éluées par un gradient linéaire de NaCI variant de 0 à 500 mM et appliqué au tampon de lavage de la colonne (PBS). Les formes de 26 et 25 kDa, produits de dégradation de la protéine recombinante complète de 27 kDa, éluent respectivement à environ 310 et 280 mM NaCl. Comme ces deux formes présentent une baisse d'affinité pour l'héparine par rapport à la protéine recombinante complète, il était important de connaître la région où s'effectuent les troncatures. Pour ce faire, les extrémités amino-terminales des 3 formes purifiées sur héparine-Sépharose furent microséquencés. L'analyse des 10 premiers acides aminés révéla que les 3 espèces moléculaires présentent la même séquence qui est identique à la séquence aminoterminale de la HBHA purifiée à partir de parois de BCG. Cette observation montre donc que la dégradation de la HBHA recombinante s'effectue par clivages carboxy-terminaux successifs. Ces clivages allant de pair avec une baisse d'affinité pour l'héparine montrent que les acides aminés carboxy-terminaux de la HBHA sont directement impliqués dans la reconnaissance des polysaccharides sulfatés. La grande teneur en lysines dans les zones répétées carboxyterminales de la HBHA plaide par ailleurs pour une interaction électrostatique entre les charges positives des lysines et les charges négatives portées par les résidus sulfatés de l'héparine.

Le rôle des lysines dans les interactions protéines-sucres sulfatés est d'ailleurs documenté (18).

La forme de 19 kDa observée après protéolyse de la HBHA recombinante pourrait donc correspondre à une HBHA ayant perdu ses séquences répétées carboxy-terminales et correspondant à une perte de 39 acides aminés. Dans cette hypothèse, la chaîne polypeptidique de la HBHA perdrait une masse d'environ 3,8 kDa et passerait ainsi de 21,3 à 17,5 kDa. Le poids moléculaire apparent de la HBHA recombinante après protéolyse dans un lysat de E. coli XL1-Blue est tout à fait compatible avec cette hypothèse, il est aussi intéressant de remarquer que la migration électrophorétique aberrante de la HBHA et attribuée à ses séquences répétées carboxy-terminales, n'est plus observée avec la forme dégradée de 19 kDa qui ne fixe plus l'héparine. Cette dernière observation renforce donc l'hypothèse selon laquelle la HBHA recombinante de 19 kDa correspondrait à une HBHA ayant perdu ses séquences répétées carboxy-terminales. Comme l'anticorps monoclonal 3921 E4 ne reconnaît plus la HBHA recombinante de 19 kDa, cela suggère que son épitope serait localisé dans les séquences répétées carboxy-terminales.

### Réactivité immunitaire vis-à-vis d'HBHA par des antisérums des tuberculeux

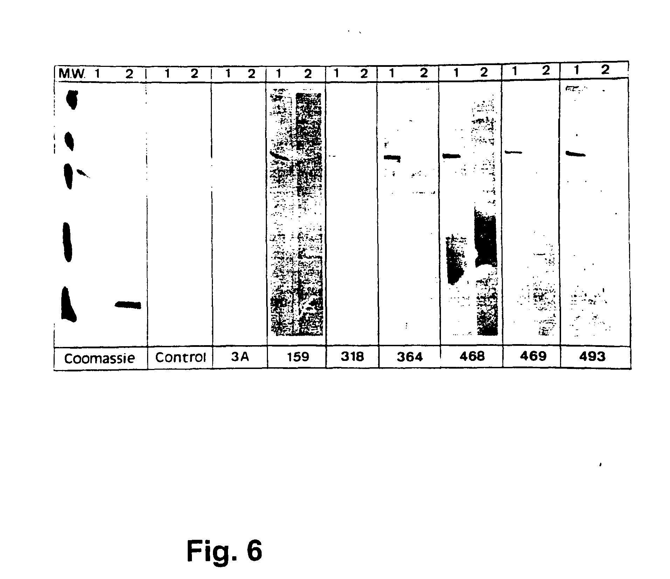

Pour savoir si HBHA est capable d'induire une réponse immunitaire chez l'homme, des analyses d'immunoempreintes ont été réalisées en utilisant de l'HBHA purifiée et des antisérums humains provenant de tuberculeux. Cinq microgrammes de la protéine ont été soumis à l'électrophorèse en utilisant un gel de polyacrylamide-SDS à 15 %, puis transférés sur des membranes de nitrocelluloses sondées ultérieurement avec les sérums dilués 100 fois provenant de 7 patients différents atteints de tuberculose évolutive (voir figure 6, pistes 3A à 493) (piste 1, HBHA purifiées, piste 2, protéine non pertinente), ainsi que le sérum d'un sujet sain (piste témoin). Les pistes de gauche contiennent les protéines purifiées (pistes 1 et 2) et les marqueurs de poids moléculaire (voir PM) ont été colorés au bleu de Coomassie après SDS-PAGE.

Les résultats présentés à la fig. 6 montrent que tous les sérums des tuberculeux contiennent des anticorps anti-HBHA, alors que le sérum de l'individu sain ne contenait pas de tels anticorps. Ces résultats indiquent que l'HBHA exposée à la surface est immunogène pendant l'évolution de la tuberculose humaine et que la présence d'anticorps anti-HBHA permet de déterminer la présence d'infections mycobactériennes.

La modification post-traductionnelle portée par la HBHA native est un déterminant antigénique majeur chez les patients tuberculeux :

La HBHA recombinante produite par E. coli fut analysée par immunoblot en utilisant les séras de patients tuberculeux. Comparativement aux signaux de détection obtenus avec de la HPHA native, ces réactifs immunologiques ont reconnu très faiblement la protéine recombinante, indiquant donc que la modification post-traductionnelle de la HBHA porte un ou des épitopes majeurs. Ces séras se sont avérés incapables de détecter la HBHA recombinante de 19 kDa.

L'invention concerne donc l'utilisation d'une séquence peptidique ou de la protéine de l'invention, et particulièrement une ou plusieurs régions immunogènes de cette séquence ou protéine, dans le diagnostic d'infections mycobactériennes, notamment par la mise en évidence de la présence d'anticorps anti-HBHA dans des fluides biologiques. De façon générale, une séquence peptidique ou la protéine ou un-polypeptide recombinant de préférence modifié selon l'invention est lié à un support et incubé avec des fluides biologiques susceptibles de contenir des anticorps anti-HBHA. Les anticorps anti-HBHA liés au polypeptide de l'invention sont ensuite mis en évidence soit par exemple avec des anticorps marqués dirigés contre les anticorps anti-HBHA, soit avec des anticorps non marqués dirigés contre les anticorps anti-HBHA et des anticorps marqués par exemple par une enzyme de type phosphatase alcaline ou peroxidase, ou de la biotine dirigés contre les anticorps non marqués.

De façon détaillée, la protéine complète ou un polypeptidide recombinant de l'invention peut être utilisée mais on peut également utiliser une ou plusieurs régions immunogènes de cette protéine qui peuvent être déterminées par des techniques dites d'« épitope mapping » bien connues de la personne versée dans l'art. Par exemple, en vue de cartographier les épitopes B et T présents dans la molécule de HBHA complète, on a recours à la méthode des profils d'hydrophobicité (19). La prédiction informatisée de déterminants antigéniques pour cellules B est aussi utilisée (20).

Le polypeptide choisi est adsorbé sur un support de type plaque de microtitration, canule, microbille ou autre. La fixation du polypeptide est effectuée en utilisant des techniques connues de la personne versée dans l'art. De façon préférée, le support utilisé est une plaque de microtitration. Le polypeptide est donc dilué dans un tampon carbonate basique et distribué dans les puits. Après une incubation de quelques heures à température ambiante, on effectue plusieurs lavages en utilisant un tampon physiologique.

Le polypeptide fixé au support est ensuite incubé avec un échantillon de fluide biologique. Plusieurs types de fluides biologiques peuvent être utilisés et obtenus à partir d'animaux ou d'humains. Plus particulièrement, le fluide biologique peut être obtenu à partir de sérum, de lymphe, de salive ou d'urine ou encore isolé à partir de tissus telles les cellules de poumons. L'incubation se fait selon les procédés habituels. Ici, les sérums de patient sont dilués et mis en contact avec la séquence peptidique fixée sur la plaque. L'incubation d'environ une heure est suivie de plusieurs lavages avec un tampon physiologique. Les anticorps anti-HBHA liés au polypeptide de l'invention sont ensuite mis en évidence soit avec des anticorps marqués dirigés contre les anticorps anti-HBHA, soit avec des anticorps non marqués dirigés contre les anticorps anti-HBHA puis avec des anticorps marqués dirigés contre les anticorps non marqués.

Le marquage des anticorps peut être radioactif mais on préférera de façon générale un autre type de marquage habituel. Les substances fluorescentes telle que l'ésithiocyanatefluoriscine ou les enzymes tels que la phosphatase alcaline, la peroxydase ou la biotine/streptavidine sont des marqueurs communément utilisés. Le choix des anticorps marqués ou non marqués dépend de l'animal duquel proviennent les liquides biologiques. S'il s'agit de liquide biologique humain, les anticorps utilisés sont dirigés contre des immunoglobulines humaines.

La liaison entre l'anticorps anti-HBHA et les anticorps marqués ou non marqués s'effectue selon les techniques habituelles. Par exemple, la réaction a lieu pendant une heure à température ambiante et après plusieurs lavages, un substrat du marqueur est ajouté.

L'invention concerne également un kit permettant de détecter la présence d'anticorps anti-HBHA dans un échantillon de fluide biologique. Le kit comprend un polypeptide, ou une ou plusieurs régions immunogènes de la HBHA adsorbée sur un support et de façon optionnelle, un anticorps marqué (et si nécessaire un anticorps non marqué) ainsi que les tampons habituels et un substrat du marqueur.

### BIBLIOGRAPHIE

1. Smith, P.G., & Moss, A.R. (1994) in *Tuberculosis, Pathogenesis, Protection, and Control*, ed. Bloom, B.R. (ASM Press, Washington, DC), pp. 47-59.
2. Raviglione, M.C., Snider, D.E., & Kochi, A. (1995) *J*. *Am. Med. Assoc.* 273, 220-226.
3. Noordeen, S.K., Bravo, L.L., & *Sundaresan,* T.K. (1992) *Bull.* WHO 70, 7.
4. Horsburgh, C.R. (1991) *N. Engl. J. Med.* 324, 1332.
5. Barnes, P.F., Bloch, A.B., Davidson, P.T., & Snider, D.E. (1991) N. *Engl. J. Med.* 324, 1644.
6. Bloch, A.B., Cauthen, G.M., Onorato, I.M., Dansbury, K.G., Kelly, G.D., Driver, C.R., & Snider, D.E. (1994) *J. Am. Med. Assoc.* 271, 665-671.
7. Bloom, B.R., & Murray, C.L. (1992) *Science* 257, 1005-1064.
8. Riley, L.W. (1995) *Trends Microbiol.* 3, 27-31.
9. Shepard, C.C. (1957) *J. Exp. Med.* 105, 39-48.
10. Schlesinger, L.S., Bellinger-Kawahara, C.G., Payne, N.R., & Horwitz, M. A. (1990) *J. Immunol.* 144, 2771-2780.
11. Schlesinger, L.S., & Horwitz, M.A. (1990) *J. Clin. Invest.* 85, 1304-1314.
12. Hirsch, C.S., Ellner, J.J., Russell, D.G., & Rich, E.A. (1994) *J*. *Immunol.* 152, 743-753.
13. Laemmli, U.K. (1970) *Nature (London)* 227, 680-685.
14. Rouse, D.A., Morris, S.L., Karpus, A.B., Mackall, J.C., Probst, P.G., & Chaparas, S.D. (1991) *Infect. Immun.* 59, 2595-2600.
15. Wiker, H.G., & Harboe, M. (1992) *Microbiol. Rev.* 56, 648-661.
16. Kremer, L., Baulard, A., Estaquier, J., Poulain-Godefroy, O., & Locht, C. (1995) *Mol. Microbiol.* 17, 913-922.
17. Sambrook, J., Fritsch, E.F., and Maniatis, T. (1989) *Molecular Cloning. A Laboratory Manual.* Cold Spring Harbor, New York: Cold Spring Harbor Laboratory Press.
18. A.D. CARDIN & H.J.R. WEINTRAUB, 1989, Molecular modeling of protein glycoaminoglycans interactions, Arteriosclerosis, 9, 21-32.
19. T.P. Hopp, 1989, Use of hydrophilicity plotting procedures to identify protein antigenic segments and other interaction sites, Methods in Enzymology, 178, 571-585.
20. V. Krchnak, O. Mach and A. Maly, 1989, Computer prediction of B-cell determinants from protein amino acid sequences based on incidence of β turns, Methods in Enzymology, 178, 586-611.
21. F. Menozzi et al., 1995, A heparin-binding hemagglutinin in mycobacteria, Abstracts of the General Meeting of the American Society for Microbiology, 193, Abstract B-159.
22. M.J. Brennan et al., 1995, Identification of a heparin-binding mycobacterial hemagglutinin, Journal of Cellular Biochemistry, Supplement 198, 66, B3-104.
23. F.D. Menozzi et al. 1996 Identification of a heparin-binding Hemagglutinin Present in Mycobacteria, The Journal of Experimental Medicine, 184, 993-1001.

## Revendications

1. Antigène protéique mycobactérien choisi dans le groupe constitué :
a) de la partie C-terminale du polypeptide HBHA de la figure 10,
b) de la séquence peptidique des 50 derniers acides aminés du polypeptide HBHA de la figure 10, et
c) d'une séquence peptidique des 30 à 50 derniers acides aminés du polypeptide HBHA de la figure 10,
ledit antigène permettant l'adhérence des mycobactéries aux glucides sulfatés des cellules épithéliales.

2. Antigène selon la revendication 1, **caractérisé en ce que** le polypeptide permettant l'adhérence est compris dans la séquence suivante :
KKAAPAKKAAPAKKAAPAKKAAAKKAPAKKAAAKKVTQK.
ou toute portion de cette séquence permettant l'adhérence de mycobactéries à des cellules hôtes.

3. Antigène selon la revendication 1, susceptible d'être obtenu à partir d'une mycobactérie, et en particulier *M. bovis* BCG.

4. Antigène selon l'une des revendications précédentes, **caractérisé en ce qu'**il est reconnu par les anticorps monoclonaux 3921 E4 et 4057 D2.

5. Polypeptide recombinant, susceptible d'être obtenu par expression dans un hôte cellulaire, d'un polynucléotide codant pour la partie C-terminale du polypeptide HBHA de la figure 10, ou les 50 derniers acides aminés du polypeptide HBHA de la figure 10 ou les 30 à 50 derniers acides aminés du polypeptide HBHA de la figure 10, et constitutif d'un antigène mycobactérien HBHA permettant l'adhérence des mycobactéries aux glucides sulfatés des cellules épithéliales.

6. Polypeptide recombinant selon la revendication 5, **caractérisé en ce que** la séquence polynucléotidique est obtenue à partir de M. *bovis* BCG ou *M. tuberculosis.*

7. Polypeptide recombinant selon l'une quelconque des revendications 5 à 6, **caractérisé en ce qu'**il est reconnu par l'anticorps monoclonal 3921 E4 et non reconnu par l'anticorps monoclonal 4057 D2.

8. Polypeptide recombinant selon l'une quelconque des revendications 5 à 7, compris dans les 50 derniers acides aminés de l'extrémité C-terminale du polypeptide HBHA de la Figure 10.

9. Polypeptide recombinant selon la revendication 7, **caractérisé en ce que** l'hôte cellulaire est *E*. *Coli.*

10. Composition immunogène contre les infections mycobactériennes contenant à titre de principe actif un antigène protéique selon l'une des revendications 1 à 4 ou un polypeptide selon l'une des revendications 5 à 8.

11. Réactif, pour la détection d'anticorps anti-HBHA dans un fluide biologique, choisi dans le groupe constitué :
a) de la partie C-terminale du polypeptide HBHA de la figure 10,
b) de la séquence peptidique des 50 derniers acides aminés du polypeptide HBHA de la figure 10, et
c) d'une séquence peptidique des 30 à 50 derniers acides aminés du polypeptide HBHA de la figure 10,
d) d'un antigène selon l'une quelconque des revendications 2 à 4, et
e) d'un polypeptide recombinant selon l'une quelconque des revendications 5 à 9.

12. Trousse pour le diagnostic sérologique des infections aux mycobactéries comprenant au moins :
a) un réactif selon la revendication 11, ledit réactif étant couplé ou adsorbé sur un support ;
b) un anticorps anti-anticorps, modifié de telle façon qu'un signal de détection puisse lui être couplé.

13. Trousse selon la revendication 12, **caractérisée en ce que** l'anticorps anti-anticorps est spécifique des immunoglobulines humaines.

14. Trousse selon la revendication 12 ou 13 dans laquelle l'anticorps anti-anticorps est marqué directement ou indirectement, soit par une substance marqueur, soit par une enzyme qui par transformation de son substrat émettra un signal de marquage.

15. Polynucléotide **caractérisé en ce que** sa séquence code pour un antigène selon la revendication 1.

16. Polynucléotide selon la revendication 15, **caractérisé en ce que** sa séquence est comprise dans une séquence codant pour le polypeptide suivant :
KKAAPAKKAAPAKKAAPAKKAAAKKAPAKKAAAKKVTQK.
ou toute portion de cette séquence peptidique permettant l'adhérence de mycobactéries à des cellules hôtes.

17. Vecteur recombinant **caractérisé en ce qu'**il comprend une séquence nucléotidique selon l'une quelconque des revendications 15 à 16.

18. Hôte cellulaire recombinant **caractérisé en ce qu'**il contient le vecteur recombinant de la revendication 17.

19. Hôte cellulaire recombinant selon la revendication 18, **caractérisé en ce que** ledit hôte est une mycobactérie.

20. Hôte cellulaire recombinant selon l'une quelconque des revendications 18 à 19, **caractérisé en ce que** ledit hôte cellulaire est *M*. *bovis* BCG.

21. Hôte selon l'une quelconque des revendications 18 à 20, **caractérisé en ce que** ladite séquence nucléotidique est surexprimée par ledit hôte.

## Claims

1. A protein mycobacterial antigen selected in the group consisting of:
a) the C-terminal portion of the HBHA polypeptide sequence shown in Figure 10,
b) the peptide sequence of the last 50 amino acids of the HBHA polypeptide sequence shown in Figure 10, and
c) a peptide sequence of the last 30 to 50 amino acids of the HBHA polypeptide sequence shown in Figure 10,
said antigen enabling the adhesion of mycobacteria to the sulphated glucides of epithelial cells.

2. Antigen according to claim 1, **characterized in that** the peptide sequence enabling the adhesion is comprised in the following sequence:
KKAAPAKKAAPAKKAAPAKKAAAKKAPAKKAAAKKVTQK
or any portion of this sequence enabling mycobacteria to adhere to host cells.

3. Antigen according to claim 1, obtainable from a mycobacterium and particularly M. *bovis* BCG.

4. Antigen according to any one of the preceding claims, **characterized in that** it is recognised by the monoclonal antibodies 4057 D2 and 3921 E4.

5. A recombinant polypeptide sequence which is obtainable by expression in a host cell of a polynucleotide encoding the C-terminal portion of the HBHA polypeptide sequence shown in Figure 10, the peptide sequence of the last 50 amino acids of the HBHA polypeptide sequence shown in Figure 10, or the peptide sequence of the last 30 to 50 amino acids of the HBHA polypeptide sequence shown in Figure 10 and constituting an HBHA mycobacterial antigen enabling the adhesion of mycobacteria to the sulphated glucides of epithelial cells.

6. Recombinant polypeptide according to claim 5, **characterized in that** the polynucleotide sequence is obtained from M. *bovis* BCG or M. *tuberculosis.*

7. Recombinant polypeptide according to any one of claims 5 to 6, **characterized in that** it is recognised by the monoclonal antibody 3921 E4 and not recognised by the monoclonal antibody 4057 D2.

8. Recombinant polypeptide according to any one of claims 5 to 7, comprised in the last 50 amino acids of the C-terminal extremity of the HBHA polypeptide of Figure 10.

9. Recombinant polypeptide according to claim 7, **characterized in that** the host cell is *E*. *coli.*

10. Immunogenic composition against mycobacterial infections containing, as an active principle, a protein antigen according to any one of claims 1 to 4 or a polypeptide according to any one of claims 5 to 8.

11. Reactant for detecting an anti-HBHA antibody in a biological fluid, selected from the group consisting of:
a) the C-terminal portion of the HBHA polypeptide sequence shown in Figure 10;
b) the peptide sequence of the last 50 amino acids of the HBHA polypeptide sequence shown in Figure 10;
c) a peptide sequence of the last 30 to 50 amino acids of the HBHA polypeptide sequence shown in Figure 10;
d) an antigen according to any one of claims 2 to 4; and
e) a recombinant polypeptide according to any one of claims 5 to 9.

12. A kit for serological diagnosis of mycobacterial infections comprising at least:
a) a reactant according to claim 11, said reactant being coupled to or adsorbed on a support;
b) an anti-antibody antibody, modified such that a detection signal can be coupled thereto.

13. A kit according to claim 12, **characterized in that** the anti-antibody antibody is specific for human immunoglobulins.

14. A kit according to claim 12 or claim 13, in which the anti-antibody antibody is directly or indirectly labelled, either using a labelling substance, or by an enzyme which emits a labelling signal by virtue of a transformation of its substrate.

15. A polynucleotide, **characterized in that** its sequence encodes an antigen according to claim 1.

16. Polynucleotide according to claim 15, **characterized in that** said sequence is comprised in a sequence encoding the following polypeptide:
KKAAPAKKAAPAKKAAPAKKAAAKKAPAKKAAAKKVTQK
or any portion of said peptide sequence enabling mycobacteria to adhere to host cells.

17. Recombinant vector, **characterized in that** it comprises a nucleotide sequence according to any one of claims 15 to 16.

18. Recombinant host cell, **characterized in that** it contains a recombinant vector according to claim 17.

19. Recombinant host cell according to claim 18, **characterized in that** said host is a mycobacterium.

20. Recombinant host cell according to any one of claims 18 to 19, **characterized in that** said host is M. *bovis* BCG.

21. Recombinant host cell according to any one of claims 18 to 20, **characterized in that** said nucleotide sequence is overexpressed by said host.

## Patentansprüche

1. Mycobakterielles Proteinantigen ausgewählt aus der Gruppe bestehend aus:
a) dem C-terminalen Teil des Polypeptids HBHA der Figur 10,
b) der Peptidsequenz der 50 letzten Aminosäuren des Polypeptids HBHA der Figur 10 und
c) einer Peptidsequenz der 30 bis 50 letzten Aminosäuren des Polypeptids HBHA der Figur 10,
wobei das Antigen die Anlagerung der Mycobakterien an sulfathaltige Kohlenhydrate von Epithelzellen erlaubt.

2. Antigen gemäß Anspruch 1, **dadurch gekennzeichnet, dass** das Polypeptid, das die Anlagerung ermöglicht, in der folgenden Sequenz umfasst ist:
KKAAPAKKAAPAKKAAPAKKAAAKKAPAKKAAAKKVTQK
oder jeder Teil dieser Sequenz, der die Anlagerung der Mycobakterien an die Wirtszellen erlaubt.

3. Antigen gemäß Anspruch 1, das ausgehend von einem Mycobakterium erhalten werden kann, und insbesondere aus M. *bovis* BCG.

4. Antigen gemäß einem der vorhergehenden Ansprüche, **dadurch gekennzeichnet, dass** es von den monoclonalen Antikörpern 3921 E4 und 4057 D2 erkannt wird.

5. Rekombinantes Polypeptid, das erhalten werden kann durch Expression eines Polynucleotids, das den C-terminalen Teil des Polypeptids HBHA der Figur 10 codiert oder die 50 letzten Aminosäuren des Polypeptids HBHA der Figur 10 oder die 30 bis 50 letzten Aminosäuren des Polypeptids HBHA der Figur 10, in einer Wirtzelle und das ein mycobakterielles Antigen HBHA darstellt, das die Anlagerung der Mycobakterien an sulfathaltige Kohlenhydrate von Epithelzellen erlaubt.

6. Rekombinantes Polypeptid gemäß Anspruch 5, **dadurch gekennzeichnet, dass** die Polynucleotidsequenz ausgehend von *M. bovis* BCG oder *M. tuberculosis* erhalten wird.

7. Rekombinantes Polypeptid gemäß einem der Ansprüche 5 bis 6, **dadurch gekennzeichnet, dass** es von dem monoclonalen Antikörper 3921 E4 erkannt wird und von dem monoclonalen Antikörper 4057 D2 nicht erkannt wird.

8. Rekombinantes Polypeptid gemäß einem der Ansprüche 5 bis 7, enthalten in den 50 letzten Aminosäuren des C-terminalen Endes des Polypeptids HBHA der Figur 10.

9. Rekombinantes Polypeptid gemäß Anspruch 7, **dadurch gekennzeichnet, dass** die Wirtszelle *E. coli.* ist.

10. Immunogene Zusammensetzung gegen mycobakterielle Infektionen, die als Wirkstoff ein Proteinantigen gemäß einem der Ansprüche 1 bis 4 enthält oder ein Polypeptid gemäß einem der Ansprüche 5 bis 8.

11. Reagens zum Nachweis von Anti-HBHA-Antikörpern in einer biologischen Flüssigkeit, ausgewählt aus der Gruppe bestehend aus:
a) dem C-terminalen Teil des Polypeptids HBHA der Figur 10
b) der Peptidsequenz der 50 letzten Aminosäuren des Polypeptids HBHA der Figur 10 und
c) einer Peptidsequenz der 30 bis 50 letzten Aminosäuren des Polypeptids HBHA der Figur 10,
d) einem Antigen gemäß einem der Ansprüche 2 bis 4 und
e) einem rekombinanten Polypeptid gemäß einem der Ansprüche 5 bis 9.

12. Kit zur serologischen Diagnose von mycobakteriellen Infektionen, mindestens umfassend:
a) ein Reagens gemäß Anspruch 11, wobei das Reagens an einen Träger gekoppelt oder adsorbiert ist;
b) einen gegen Antikörper gerichteten Antikörper, der in einer solchen Weise modifiziert ist, dass ein Nachweissignal an ihn gekoppelt werden kann.

13. Kit gemäß Anspruch 12, **dadurch gekennzeichnet, dass** der gegen Antikörper gerichtete Antikörper für menschliche Immunglobuline spezifisch ist.

14. Kit gemäß Anspruch 12 oder 13, wobei der gegen Antikörper gerichtete Antikörper direkt oder indirekt markiert ist, entweder durch eine Markersubstanz oder durch ein Enzym, das durch Umsetzung seines Substrates ein Markersignal erzeugen wird.

15. Polynucleotid, **dadurch gekennzeichnet, dass** seine Sequenz ein Antigen gemäß Anspruch 1 codiert.

16. Polynucleotid gemäß Anspruch 15, **dadurch gekennzeichnet, dass** seine Sequenz in einer Sequenz enthalten ist, die für folgendes Polypeptid codiert:
KKAAPAKKAAPAKKAAPAKKAAAKKAPAKKAAAKKVTQK
oder jeder Teil dieser Peptidsequenz, der die Anlagerung der Mycobakterien an Wirtszellen erlaubt.

17. Rekombinanter Vektor, **dadurch gekennzeichnet, dass** er eine Nucleotidsequenz gemäß einem der Ansprüche 15 bis 16 umfasst.

18. Rekombinante Wirtszelle, **dadurch gekennzeichnet, dass** sie den rekombinanten Vektor gemäß Anspruch 17 enthält.

19. Rekombinante Wirtszelle gemäß Anspruch 18, **dadurch gekennzeichnet, dass** der Wirt ein Mycobakterium ist.

20. Rekombinante Wirtszelle gemäß einem der Ansprüche 18 bis 19, **dadurch gekennzeichnet, dass** die Wirtszelle M. *bovis* BCG ist.

21. Wirt gemäß einem der Ansprüche 18 bis 20, **dadurch gekennzeichnet, dass** die Nucleotidsequenz durch den Wirt überexprimiert wird.


### SEQUENCE LISTING

<110> INSTITUT PASTEUR DE LILLE INSTITUT NATIONAL DE LA SANTE ET DE LA RECHERCHE M
<120> IDENTIFICATION AND CLONING OF A MYCOBACTERIAL ANTIGEN CORRESPONDING TO A HEPARIN-BINDING HAEMAGGLUTININ
<130> B3168AAA - I.P.L./INSERM
<140> EP 97925109.7
   <141> 1997-05-20
<150> FR 96 06168
   <151> 1996-05-17
<160> 20
<170> PatentIn Ver. 2.1
<210> 1
   <211> 39
   <212> PRT
   <213> Artificial Sequence
<220>
   <223> Description of Artificial Sequence: peptide sequence comprising a region involved in interactions with sulphated glycoconjugates and in heparin binding
<400> 1
   Lys Lys Ala Ala Pro Ala Lys Lys Ala Ala Pro Ala Lys Lys Ala Ala
     1               5                  10                  15
   Pro Ala Lys Lys Ala Ala Ala Lys Lys Ala Pro Ala Lys Lys Ala Ala
                20                  25                  30
   Ala Lys Lys Val Thr Gln Lys
            35
<210> 2
   <211> 10
   <212> PRT
   <213> Artificial Sequence
<220>
   <223> Description of Artificial Sequence: peptide S1441
<400> 2
   Lys Ala Glu Gly Tyr Leu Glu Ala Ala Thr
     1               5                  10
<210> 3
   <211> 18
   <212> PRT
   <213> Artificial Sequence
<220>
   <223> Description of Artificial Sequence: peptide S1443
<400> 3
   Xaa Glu Gly Tyr Val Asp Gln Ala Val Glu Leu Thr Gln Glu Ala Leu
    1               5                  10                  15
   Gly Lys
<210> 4
   <211> 9
   <212> PRT
   <213> Artificial Sequence
<220>
   <223> Description of Artificial Sequence: peptide S1446
<400> 4
   Xaa Gln Glu Xaa Leu Pro Glu Xaa Leu
    1               5
<210> 5
   <211> 7
   <212> PRT
   <213> Artificial Sequence
<220>
   <223> Description of Artificial Sequence: Peptide S1447
<400> 5
   Phe Thr Ala Glu Glu Leu Arg
    1               5
<210> 6
   <211> 17
   <212> DNA
   <213> Artificial Sequence
<220>
   <223> Description of Artificial Sequence: Oligonucleotide originated from the S1441 peptide (oligo S1441)
<400> 6
   aaggcsgagg gstacct                                              17
<210> 7
   <211> 17
   <212> DNA
   <213> Artificial Sequence
<220>
   <223> Description of Artificial Sequence: Oligonucleotide originated from the S1441 peptide (reverse oligo S1441)
<400> 7
<210> 8
   <211> 17
   <212> DNA
   <213> Artificial Sequence
<220>
   <223> Description of Artificial Sequence: Oligonucleotide originated from the S1443 peptide (oligo S1443)
<400> 8
   gaccaggcsg tsgagct 17
<210> 9
   <211> 17
   <212> DNA
   <213> Artificial Sequence
<220>
   <223> Description of Artificial Sequence: Oligonucleotide originated from the S1443 peptide (reverse oligo S1443)
<400> 9
   agctcsacsg cctggtc 17
<210> 10
   <211> 21
   <212> DNA
   <213> Artificial Sequence
<220>
   <223> Description of Artificial Sequence: Oligonucleotide named HBHASeq1 and used for sequencing the gene coding for HBHA
<400> 10
   agccggtaca acgagctggt c 21
<210> 11
   <211> 21
   <212> DNA
   <213> Artificial Sequence
<220>
   <223> Description of Artificial Sequence: Oligonucleotide named HBHA Seqlinv and used for sequencing the gene coding for HBHA
<400> 11
   gaccagctcg ttgtaccggc t 21
<210> 12
   <211> 19
   <212> DNA
   <213> Artificial Sequence
<220>
   <223> Description of Artificial Sequence: Oligonucleotide named HBHASeq2 and used for sequencing the gene coding for HBHA
<400> 12
   catccaacac gtcgactcc 19
<210> 13
   <211> 19
   <212> DNA
   <213> Artificial Sequence
<220>
   <223> Description of Artificial Sequence: Oligonucleotide named HBHA Seq3 and used for sequencing the gene coding for HBHA
<400> 13
   ttgatgtcat caatgttcg 19
<210> 14
   <211> 19
   <212> DNA
   <213> Artificial Sequence
<220>
   <223> Description of Artificial Sequence: Oligonucleotide named HBHA Seq4 and used for sequencing the gene coding for HBHA
<400> 14
   cgtggaccag gcggtggag 19
<210> 15
   <211> 21
   <212> DNA
   <213> Artificial Sequence
<220>
   <223> Description of Artificial Sequence: Oligonucleotide named HBHA Seq 5 and used for sequencing the gene coding for HBHA
<400> 15
   gacgatcagg aggtttcccc g 21
<210> 16
   <211> 24
   <212> DNA
   <213> Artificial Sequence
<220>
   <223> Description of Artificial Sequence: Oligonucleotide named reverse primer and used for sequencing the gene coding for HBHA
<400> 16
   agcggataac aatttcacac agga 24
<210> 17
   <211> 149
   <212> DNA
   <213> Artificial Sequence
<220>
   <223> Description of Artificial Sequence: nucleotide sequence and amino sequence of a fragment of HBHA deduced from a PCR fragment of chromosomal BCG DNA
<220>
   <221> CDS
   <222> (1)..(147)
<400> 17
   aag gcc gag ggc tac ctc gag gcc gcg act agc cgg tac aac gag ctg    48
   Lys Ala Glu Gly Tyr Leu Glu Ala Ala Thr Ser Arg Tyr Asn Glu Leu
     1               5                  10                  15
   gtc gag cgc ggt gag gcc gct cta gag cgg ctg cgc agc cag cag agc    96
   Val Glu Arg Gly Glu Ala Ala Leu Glu Arg Leu Arg Ser Gln Gln Ser
                20                  25                  30
   ttc gag gaa gtg tcg gcg ccc gcc gaa ggc tac gtg gac cag gcg gtc   144
   Phe Glu Glu Val Ser Ala Pro Ala Glu Gly Tyr Val Asp Gln Ala Val
            35                  40                  45
   gag ct                                                             149
   Glu
<210> 18
   <211> 49
   <212> PRT
   <213> Artificial Sequence
   <223> Description of Artificial Sequence: nucleotide sequence and amino sequence of a fragment of HBHA deduced from a PCR fragment of chromosomal BCG DNA
<400> 18
   Lys Ala Glu Gly Tyr Leu Glu Ala Ala Thr Ser Arg Tyr Asn Glu Leu
     1               5                  10                  15
   Val Glu Arg Gly Glu Ala Ala Leu Glu Arg Leu Arg Ser Gln Gln Ser
                20                  25                  30
   Phe Glu Glu Val Ser Ala Pro Ala Glu Gly Tyr Val Asp Gln Ala Val
            35                  40                  45
   Glu
<210> 19
   <211> 1097
   <212> DNA
   <213> Artificial Sequence
<220>
   <223> Description of Artificial Sequence: DNA sequence of the BCG gene coding for HBHA
<220>
   <221> CDS
   <222> (331)..(924)
   <223> CDS from 811 to 828, from 829 to 846, from 847 to 864, from 865 to 885 and from 895 to 915 : peptide which may be particularly involved in interactions with sulphated glycoconjugates
<400> 19
   cggctggcgg gtaatcaaac ctgaaggaca gtcatctggg tgaggtcgac cgcaggctga 60
   tccagccgat cggccggcgc tggccaacag cgactccgtc gatgacgtgc agcaaaggag 120
   acatgtagtg accggatcag ctgggcctga catctacgaa ctcgaccgac aaccgacccg 180
   acgatcagga ggtttccccg gcaagtcgcg tgccatgtca atccgcgggt cttgactagt 240
   cctccctgga ggagccgacg cttgccccaa cgtccagacc aaagatgtaa gaacgccgat 300
   atcagaaaat agttaatgaa aggaataccc atg gct gaa aac tcg aac att gat  354
                                    Met Ala Glu Asn Ser Asn Ile Asp
                                    1               5
   gac atc aag gct ccg ttg ctt gcc gcg ctt gga gcg gcc gac ctg gcc   402
   Asp Ile Lys Ala Pro Leu Leu Ala Ala Leu Gly Ala Ala Asp Leu Ala
       10                  15                  20
   ttg gcc act gtc aac gag ttg atc acg aac ctg cgt gag cgt gcg gag   450
   Leu Ala Thr Val Asn Glu Leu Ile Thr Asn Leu Arg Glu Arg Ala Glu
   25                  30                  35                  40
   gag act cgt acg gac acc cgc agc cgg gtc gag gag agc cgt gct cgc   498
   Glu Thr Arg Thr Asp Thr Arg Ser Arg Val Glu Glu Ser Arg Ala Arg
                   45                  50                  55
   ctg acc aag ctg cag gaa gat ctg ccc gag cag ctc acc gag ctg cgt   546
   Leu Thr Lys Leu Gln Glu Asp Leu Pro Glu Gln Leu Thr Glu Leu Arg
               60                  65                  70
   gag aag ttc acc gcc gag gag ctg cgt aag gcc gcc gag ggc tac ctc   594
   Glu Lys Phe Thr Ala Glu Glu Leu Arg Lys Ala Ala Glu Gly Tyr Leu
           75                  80                  85
   gag gcc gcg act agc cgg tac aac gag ctg gtc gag cgc ggt gag gcc   642
   Glu Ala Ala Thr Ser Arg Tyr Asn Glu Leu Val Glu Arg Gly Glu Ala
       90                  95                  100
   gct cta gag cgg ctg cgc agc cag cag agc ttc gag gaa gtg tcg gcg   690
   Ala Leu Glu Arg Leu Arg Ser Gln Gln Ser Phe Glu Glu Val Ser Ala
   105                 110                 115                 120
   ccc gcc gaa ggc tac gtg gac cag gcg gtg gag ttg acc cag gag gcg    738
   Pro Ala Glu Gly Tyr Val Asp Gln Ala Val Glu Leu Thr Gln Glu Ala
                   125                 130                 135
   ttg ggt acg gtc gca tcg cag acc cgc gcg gtc ggt gag cgt gcc gcc    786
   Leu Gly Thr Val Ala Ser Gln Thr Arg Ala Val Gly Glu Arg Ala Ala
               140                 145                 150
   aag ctg gtc ggc atc gag ctg cct aag aag gct gct ccg gcc aag aag    834
   Lys Leu Val Gly Ile Glu Leu Pro Lys Lys Ala Ala Pro Ala Lys Lys
           155                 160                 165
   gcc gct ccg gcc aag aag gcc gct ccg gcc aag aag gcg gcg gcc aag    882
   Ala Ala Pro Ala Lys Lys Ala Ala Pro Ala Lys Lys Ala Ala Ala Lys
       170                 175                 180
   aag gcg ccc gcg aag aag gcg gcg gcc aag aag gtc acc cag            924
   Lys Ala Pro Ala Lys Lys Ala Ala Ala Lys Lys Val Thr Gln
   185                 190                 195
   aagtagtcgg gctccgaatc accatcgact ccgagtcgcc cacggggcga ctcggagtcg 984
   acgtgttgga tgcaaaccgc atagtctgaa tgcgtgagcc acctcgtggg taccgtcatg 1044
   ctggtattgc tggtcgccgt cttggtgaca gcggtgtacg cgtttgtgca tgc        1097
<210> 20
   <211> 198
   <212> PRT
   <213> Artificial Sequence
   <223> Description of Artificial Sequence: DNA sequence of the BCG gene coding for HBHA
<400> 20
   Met Ala Glu Asn Ser Asn Ile Asp Asp Ile Lys Ala Pro Leu Leu Ala
    1               5                  10                  15
   Ala Leu Gly Ala Ala Asp Leu Ala Leu Ala Thr Val Asn Glu Leu Ile
               20                  25                  30
   Thr Asn Leu Arg Glu Arg Ala Glu Glu Thr Arg Thr Asp Thr Arg Ser
           35                  40                  45
   Arg Val Glu Glu Ser Arg Ala Arg Leu Thr Lys Leu Gln Glu Asp Leu
       50                  55                  60
   Pro Glu Gln Leu Thr Glu Leu Arg Glu Lys Phe Thr Ala Glu Glu Leu
   65                  70                  75                  80
   Arg Lys Ala Ala Glu Gly Tyr Leu Glu Ala Ala Thr Ser Arg Tyr Asn
                   85                  90                  95
   Glu Leu Val Glu Arg Gly Glu Ala Ala Leu Glu Arg Leu Arg Ser Gln
               100                 105                 110
   Gln Ser Phe Glu Glu Val Ser Ala Pro Ala Glu Gly Tyr Val Asp Gln
           115                 120                 125
   Ala Val Glu Leu Thr Gln Glu Ala Leu Gly Thr Val Ala Ser Gln Thr
       130                 135                 140
   Arg Ala Val Gly Glu Arg Ala Ala Lys Leu Val Gly Ile Glu Leu Pro
   145                 150                 155                 160
   Lys Lys Ala Ala Pro Ala Lys Lys Ala Ala Pro Ala Lys Lys Ala Ala
                   165                 170                 175
   Pro Ala Lys Lys Ala Ala Ala Lys Lys Ala Pro Ala Lys Lys Ala Ala
               180                 185                 190
   Ala Lys Lys Val Thr Gln
           195